(19) Europäisches Patentamt
European Patent Office
Office européen des brevets

(11) Publication number: **0 447 703 A1**

(12) **EUROPEAN PATENT APPLICATION**

(21) Application number: **90303008.8**

(22) Date of filing: **20.03.90**

(51) Int. Cl.[5]: **C07D 491/044**, C07D 495/04, C07D 493/04, C07D 487/04, A61K 31/40, A61K 31/38, A61K 31/34, //(C07D491/044, 307:00,209:00),(C07D495/04, 333:00,209:00),(C07D493/04, 307:00,307:00),(C07D487/04, 209:00,209:00),(C07D495/04, 333:00,209:00)

(43) Date of publication of application:
**25.09.91 Bulletin 91/39**

(84) Designated Contracting States:
**AT BE CH DE DK ES FR GB GR IT LI LU NL SE**

(71) Applicant: **THE WELLCOME FOUNDATION LIMITED**
**Unicorn House 160 Euston Road**
**London NW1 2BP(GB)**

(72) Inventor: **Bair, Kenneth Walter**
**342 Wesley Drive**
**Chapel Hill, North Carolina 27514(US)**

(74) Representative: **Rollins, Anthony John et al**
**Group Patents & Agreements The Wellcome Foundation Ltd Langley Court**
**Beckenham Kent BR3 3BS(GB)**

(54) **Hetero polycyclic biocidal compounds, their synthesis and intermediates therefor,formulations containing them and their use in medicine.**

(57) Heterotetracyclic aromatic compounds having biocidal activity containing two hetero atoms selected from oxygen, sulphur and nitrogen and that are substituted by an aminoalkanol group of formula

$ArCH_2NHR$

or a monomethyl or monoethyl ether thereof, the compound of formula (I) including these ethers containing no more than 29 carbon atoms in total, or an ester or a salt thereof; wherein Ar is a fused tetracyclic hetero aromatic ring system of the formula:

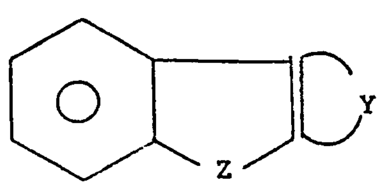

in which Z is oxygen, sulphur or a group $NR^1$ wherein $R^1$ is hydrogen, methyl or ethyl and

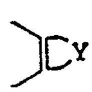

EP 0 447 703 A1

is a bicyclic aromatic ring system comprising a phenyl ring and a 5-membered ring system which contains one heteroatom $Z^1$ selected from oxygen, sulphur or a group $NR^2$ wherein $R^2$ is hydrogen, methyl or ethyl; the tetracyclic ring system being optionally substituted by one or two substituents;
and R contains not more than eight carbon atoms and is a group

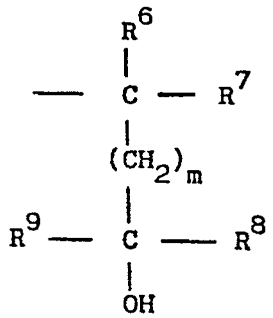

or

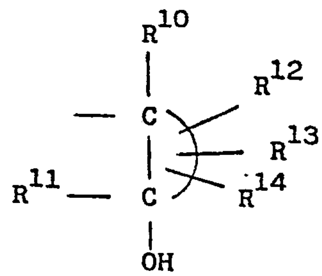

wherein m is 0 or 1;
$R^6$ is hydrogen or alkyl optionally substituted by hydroxy;
$R^7$ and $R^8$ are the same or different and each is hydrogen or alkyl;
$R^9$ is hydrogen, methyl or hydroxymethyl;

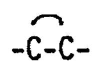

is a five-or six-membered saturated carbocyclic ring;
$R^{10}$, $R^{11}$ and $R^{12}$ are the same or different and each is hydrogen or methyl;
$R^{13}$ is hydrogen, methyl, hydroxy, or hydroxymethyl; $R^{14}$ is hydrogen, methyl, hydroxy or hydroxymethyl.

Processes for preparing the compounds, intermediates in their preparation, pharmaceutical compositions containing them and their use in medicine are also disclosed.

The present invention relates to heteropolycyclic aromatic alkanol derivatives which have been found to have biocidal activity. More specifically the invention concerns aminoalkanol derivatives containing a heteropolycyclic aromatic ring system, methods for the synthesis thereof, novel intermediates thereof, pharmaceutical formulations thereof and the use thereof as biocidal agents, particularly antitumor agents.

We have discovered a novel class of heteropolycyclic aromatic alkanol derivatives which have biocidal activity. Accordingly, in a first aspect, the present invention provides a compound of the formula (I)

$ArCH_2NHR$ (I)

or a monomethyl or monoethyl ether thereof, the compound of formula (I) including these ethers containing no more than 29 carbon atoms in total, or an ester or a salt thereof; wherein Ar is a fused tetracyclic hetero aromatic ring system of the formula:

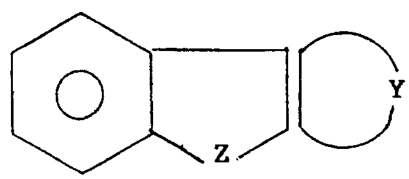

in which Z is oxygen, sulphur or a group $NR^1$ wherein $R^1$ is hydrogen, methyl or ethyl and

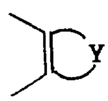

is a bicyclic aromatic ring system comprising a phenyl ring and a 5-membered ring system which contains one heteroatom $Z^1$ selected from oxygen, sulphur or a group $NR^2$ wherein $R^2$ is hydrogen, methyl or ethyl; the tetracyclic ring system being optionally substituted by one or two substituents; said substituents containing not more than four carbon atoms in total when taken together and are the same or different each being selected from halogen; cyano; $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $S(O)_nR^3$ wherein n is an integer 0,1 or 2 and $R^3$ is $C_{1-2}$ alkyl optionally substituted by hydroxy or $C_{1-2}$ alkoxy; or Ar is optionally substituted by a group $NR^4R^5$ containing not more than 5 carbon atoms wherein $R^4$ and $R^5$ are the same or different and each is a $C_{1-3}$ alkyl group or $NR^4R^5$ forms a five-or six-membered heterocyclic ring optionally containing one or two additional heteroatoms;

R contains not more than eight carbon atoms and is a group

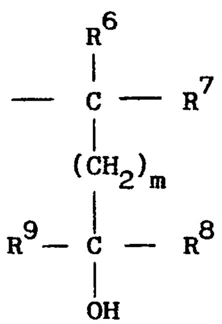

or

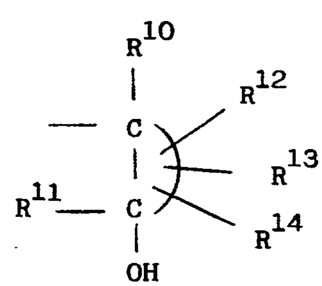

wherein m is 0 or 1;
$R^6$ is hydrogen or $C_{1-3}$ alkyl optionally substituted by hydroxy;
$R^7$ and $R^8$ are the same or different and each is hydrogen or $C_{1-3}$ alkyl;
$R^9$ is hydrogen, methyl or hydroxymethyl;

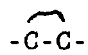

is a five-or six-membered saturated carbocyclic ring;
$R^{10}$ $R^{11}$ and $R^{12}$ are the same or different and each is hydrogen or methyl;
$R^{13}$ is hydrogen, methyl, hydroxy, or hydroxymethyl; $R^{14}$ is hydrogen, methyl, hydroxy or hydroxymethyl.

Suitably the tetracyclic aromatic ring system Ar is unsubstituted or has only one substituent. Preferably the aromatic ring is unsubstituted.

Suitably $ArCH_2NHR$ or a monomethyl or monoethyl ether thereof contains not more than 28 carbon atoms in total.

Suitably m is 0.

Suitably R is

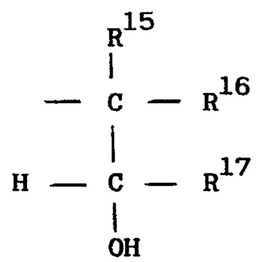

or

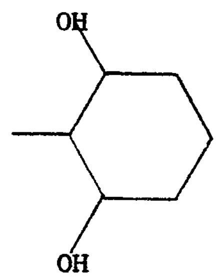

wherein $R^{15}$ is $CH_2OH$, $CH(CH_3)OH$ or $CH_2CH_2OH$,
$R^{16}$ is hydrogen, $C_{1-3}$ alkyl or $CH_2OH$,
$R^{17}$ is hydrogen or methyl.

Suitably $R^{15}$ is $CH_2OH$ or $CH(CH_3)OH$. Suitably $R^{17}$ is hydrogen, methyl, ethyl or $CH_2OH$.

Preferably R is

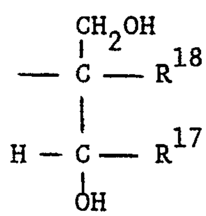

wherein $R^{17}$ is hydrogen or methyl and $R^{18}$ is hydrogen, methyl or ethyl, preferably methyl.

Tetracyclic heteroaromatic ring systems of compounds of the present invention have the following nomenclature:

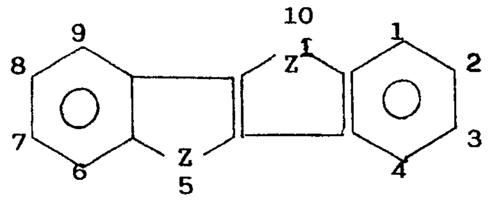

Z = S, $Z^1$ = NMe,
10-methyl-10H-[1]benzothieno[3,2-b] indol-6-yl

Z = S, $Z^1$ = NMe,
10-Methyl-10H-[1]benzothieno[3,2-b] indol-3-yl

Z = 0, $Z^1$ = NMe,
2-(10-methyl-10H-benzofuro-(3,2-b) indol-6-yl

---

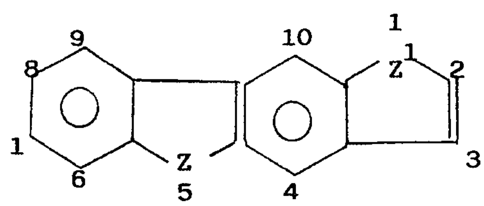

Z = 0, $Z^1$ = 0,
2-(benzofuro(5,6-b)benzofuran-4-yl,

Z = 0, $Z^1$ = 0
2-(benzofuro(5,6-b)benzofuran-8-yl-,

Z = 0, $Z^1$ = 0
2-(benzofuro(5,6-b)benzofuran-2-yl-,

---

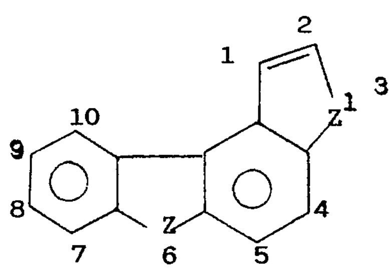

Z = 0, $Z^1$ = 0,
2-(benzofuro(5,4-b)benzofuran-2-yl-,

Z = 0, $Z^1$ = 0
2-(benzofuro(5,4-b)benzofuran-9-yl-,

Z = 0, $Z^1$ = 0,
2-(benzofuro(5,4-b)benzofuran-4-yl,

Z = 0, $Z^1$ = 0,
2-(benzofuro(5,4-b)benzofuran-8-yl-,

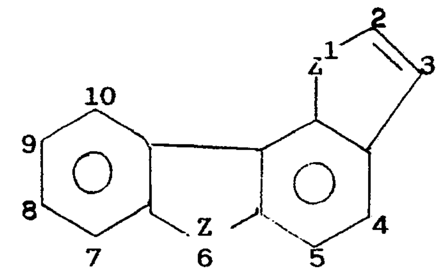

Z = S, $Z^1$ = NMe,
1-Methyl-1H-[1]benzothieno[2,3-g]indol-3-yl-

Z = O, $Z^1$ = NMe,
1-Methyl-1H-benzofuro[2,3-g]indol-3-yl-

Z = NEt, $Z^1$ - NMe,
6-Ethyl-1,6-dihydro-1-methylpyrrolo[3,2-c]carbazol-3-yl-

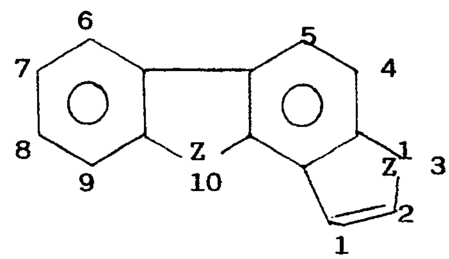

Z = S, $Z^1$ = NMe,
3-Methyl-3H-[1]benzothieno[2,3-e]indol-1-yl-

Z - NMe, $Z^1$ = S
10-Methyl-10H-thieno[3,2-a]carbazol-2-yl-

Z - NMe, $Z^1$ = S,
10-Methyl-10H-thieno[3,2-a]carbazol-4-yl-

Specific compounds within the scope of formula (I) include:
2-(((10-Methyl-10H-[1]benzothieno[3,2-b]indol-3-yl)methyl)-amino)-2-methyl-1,3-propanediol,
2-(((10-Methyl-10H[1]benzothieno[3,2-b]indol-6-yl)methyl)amino)-2-methyl-1,3-propanediol,
2-Methyl-2-(((10-methyl-10H-benzofuro(3,2-b)indol-6-yl)methyl)amino)-1,3-propanediol,
2-(((Benzofuro(5,6-b)benzofuran-4-yl)methylamino)-2-methyl-1,3-propanediol,
2-(((Benzofuro(5,6-b)benzofuran-8-yl)methyl)amino)-2-methyl-1,3-propanediol,
2-(((Benzofuro(5,6-b)benzofuran-2-yl)methyl)amino)-2-methyl-1,3-propanediol,
2-(((Benzofuro(5,4-b)benzofuran-4-yl)methyl)amino)-2-methyl-1,3-propanediol,
2-(((Benzofuro(5,4-b)benzofuran-9-yl)methyl)amino)-2-methyl-1,3-propanediol,
2-(((Benzofuro(5,4-b)benzofuran-8-yl)methyl)amino)-2-methyl-1,3-propanediol,
2-(((Benzofuro(5,4-b)benzofuran-2-yl)methyl)amino-2-methyl-1,3-propanediol, and monomethyl or monethyl ethers, esters, and addition salts thereof.

Salts included within the scope of the present invention are those of compounds of formula (I) and ethers and esters thereof.

Esters and non-pharmaceutically useful salts of the compounds of the formula (I) are useful intermediates in the preparation and purification of compounds of the formula (I) and pharmaceutically useful salts thereof, and are therefore within the scope of the present invention. Thus, salts of the compounds of the formula (I) useful in the present invention include but are not limited to those derived from inorganic acids, such as hydrochloric, hydrobromic, sulfuric and phosphoric acids, and organic acids such as isethionic (2-hydroxyethylsulfonic), maleic, malonic, succinic, salicylic, tartaric, lactic, citric, formic, lactobionic, pantothenic, methanesulfonic, ethanesulfonic, benzenesulfonic, p-toluenesulfonic, naphthalene-2-sulfonic, and ascorbic acids, and amino acids such as glycine.
Pharmacologically and pharmaceutically acceptable salts are preferred, particularly those that are soluble in solvents suitable for parenteral administration, for example, hydrochlorides, methanesulfonates and isethionates.

Esters of compounds of formula (I) are derived from acids known to those skilled in the art to be suitable for ester formation, and are conveniently those derived from $C_{1-6}$ alkanoic acids or alkanoic acid derivatives, for example acetic acid, propionic acid n-butyric acid and iso-butyric acid. The esters may be formed from all or only some of the hydroxy groups contained in the compounds of formula (I).

The compounds of formula (I) and their ethers, esters, and salts thereof may be prepared by any method known in the art for the preparation of compounds of analogous structure. Thus, the compounds of formula (I) may, for example, be prepared by any of the methods defined below.

1. The reduction of a compound Ar-CH = NR (II)
wherein Ar and R are as hereinbefore defined or an appropriately protected derivative thereof followed by deprotection where appropriate.

The conditions and reagents for such a reaction are well known to those skilled in the art, and any such conditions/reagents may be employed that will not reduce the aromatic ring system. The conversion of (II) or suitably protected derivatives thereof may be carried out by a reducing agent followed by deprotection if necessary. The reduction is conveniently carried out by a metal hydride such as lithium aluminum hydride, sodium borohydride, sodium cyanoborohydride, or by catalytic hydrogenation, conveniently by hydrogen in the presence of a metal catalyst such as palladium or platinum, or equivalent reagents as outlined by J. March, Advanced Organic Chemistry, 2nd ed., pages 819-820, McGraw Hill, New York, 1977. The reduction is suitably carried out with Ar-CH-NR in solution in an inert solvent or mixture of solvents compatible with the reducing agent, at a non-extreme temperature, for example, between 0° and 80°C, conveniently at room temperature.

In the case of lithium aluminum hydride and like reagents, suitable solvents include ethers (for example tetrahydrofuran, diethyl ether and 1,2-dimethoxyethane) optionally in the presence of a hydrocarbon cosolvent (for example toluene, benzene or hexane).

In the case of sodium borohydride and like reagents, suitable solvents include alcohols (for example ethanol, methanol or isopropanol) optionally in the presence of a hydrocarbon cosolvent (for example toluene, benzene or hexane) or an ether cosolvent (for example diethylether or tetrahydrofuran).

In the case of sodium cyanoborohydride and like reagents, suitable solvents include those described for sodium borohydride and the reduction is conveniently carried out in the presence of an acid, conveniently glacial acetic acid or ethanolic hydrochloric acid as outlined in, for example, R. Hutchins et al., Organic Preparations and Procedures International 11, 201 (1979).

In the case of catalytic hydrogenation, suitable solvents include alcohols (for example methanol and ethanol) optionally in the presence of a hydrocarbon cosolvent (for example toluene or benzene), or ether cosolvent (for example diethyl ether or tetrahydrofuran) optionally in the presence of an acid (for example glacial acetic acid or ethanolic hydrochloric acid), or glacial acetic acid.

Protected derivatives of compounds ArCH = NR are conveniently used when lithium aluminum hydride is employed as the reducing agent. Convenient protecting groups are compatible with the reducing agent utilized and are readily removed under nondestructive conditions, for example benzyl, tetrahydropyranyl and isopropylidene ethers.

It is often convenient not to isolate the compound ArCH = NR but to react a compound ArCHO with a compound $NH_2R$; wherein Ar and R are as defined in (I) and to reduce the compound ArCH = NR so formed in situ. The reaction of the compounds ArCHO and $NH_2R$ is again suitably carried out using conditions and reagents which are well known to those skilled in the art, for example in the presence of an acid, such as a sulfonic acid, i.e. p-toluenesulfonic acid, in an appropriate inert solvent, such as an aromatic hydrocarbon, suitably toluene, with azeotropic removal of water followed by treatment with the reducing agent in an appropriate solvent, suitably ethanol or methanol. Alternatively, ArCH = NR formed under equilibrium conditions in appropriate solvents can be reduced in situ with an appropriate reducing agent, suitably sodium cyanoborohydride.

The compound ArCHO may be in the form of a protected aldehyde, for example an acetal, which liberates the aldehyde functionality under the reaction conditions. In turn, ArCHO can be synthesized by reacting the appropriate aromatic polyheterocycle optionally substituted with a carbalkoxy group with a formylating agent such as that generated by the reaction between $SnCl_4$ and $Cl_2CHOCH_3$ or equivalent reagents,
for example, according to the method of A. Reiche et al., Chem. Ber. 93, 88 (1960), or with other standard formylating reagents/procedures known to the art, for example, the Gatterman-Koch reaction

($CO/HCl/AlCl_3/CuCl$), the Gatterman reaction ($HCN/HCl/ZnCl_2$), and the Vilsmeier reaction ($POCl_3$/PhN-(Me)CHO or $POCl_3/Me_2NCHO$) (J. March, vide supra pages 494-497).

The compounds ArCHO may also be prepared from an appropriate aromatic heteropolycycle substituted by a suitable functional group and converting this functional group to an aldehyde group by methods well known to those skilled in the art. Suitable functional groups include $CHBr_2$, $CH_3$, $COR^{19}$ wherein $R^{19}$ is a primary or secondary $C_{1-6}$ alkyl group, COOH or a derivative thereof such as an ester, amide or acid chloride or CN.

Where the aromatic heteropolycycle bears substituents, ArCHO may be prepared by a variety of methods known in the art of organic chemistry depending on the nature of the substituent on the ring. For example, if the substituent(s) is a halogen, the starting materials may be prepared by direct treatment of the aromatic heteropolycycle ring with a halogenating agent (e.g. $Cl_2$, $Br_2$, or $SO_2Cl_2$) or indirectly by such routes as the Sandmeyer reaction (H.H. Hodgson, Chem. Rev. 40, 251 (1947). If the substituent(s) is alkyl, the aromatic heteropolycycle may be reacted with the appropriate reagents under Friedel-Crafts reaction conditions (G.A. Olah, Friedel Crafts and Related Reactions, Vols. 1-3, Interscience, New York, NY, 1963-1965).

The compounds $NH_2R$ also may be prepared by methods known in the art, for example when R is as hereinbefore defined by the reaction of $NO_2R^{19}$ wherein $R^{19}$ is a group

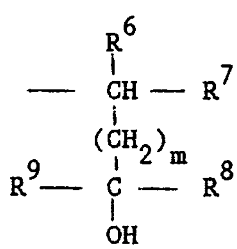

and $R^6$ to $R^9$ and m are as hereinbefore defined with an appropriate aldehyde, conveniently acetaldehyde or formaldehyde (as in B.M. Vanderbilt and H.B. Hass Ind. Eng. Chem. 32, 34 (1940)) followed by reduction (as outlined in J. March, vide supra, pages 1125-1126), conveniently by hydrogen and a metal catalyst (for example, a platinum containing catalyst) in an appropriate solvent, conveniently glacial acetic acid.

2. The reduction of a compound Ar.CO.NHR; wherein Ar and R are as hereinbefore defined and the hydroxy groups are optionally protected, followed by deprotection of the hydroxy groups where appropriate. The reduction may be carried out by standard reducing agents known for carrying out this type of reduction that will not reduce the aromatic ring system (as outlined in J. March, vide supra, page 1122), for example, a hydride reagent such as lithium aluminium hydride in an inert solvent, such as an ether, i.e. tetrahydrofuran, at a non-extreme temperature, for example, at between 0° and 100°C and conveniently at the reflux temperature of the ether.

The compound Ar.CO.NHR may be formed by the reaction of the appropriate acid (ArCOOH) or a suitable reactive acid derivative thereof as outlined in J. March, vide supra, pages 382-390) for example, an acid halide in an inert solvent, with an amine $NH_2R^1$ in which the hydroxy groups are optionally protected, for example, when the compound $NH_2R^1$ is a diol, by an isopropylidene group. The compound Ar. CO. $NHR^1$ so formed is suitably reduced in situ and deprotected if necessary to give a compound of formula (I). The compounds of the formula ArCOOH can be prepared by methods well known to those skilled in the art.

3. The reaction of a compound $ArCH_2L$ (wherein Ar is as hereinbefore defined and L is a leaving group), with a compound $NH_2R$ as hereinbefore defined. Suitable leaving groups are those defined by J. March, vide supra pages 325-331, and include halogens such as chlorine or bromine and sulfonic acid derivatives such as p-toluenesulfonate. The reaction is suitably carried out in an appropriate solvent, such as a dipolar aprotic solvent or alcohol at a non-extreme temperature, for example 50-100°. The compounds of the formula $ArCH_2L$ can be prepared by methods well known to those skilled in the art.

There is therefore provided, as a further aspect of the invention, a method for the preparation of a compound of formula (I) comprising any method known for the preparation of analogous compounds, in particular those methods defined in (1) to (3) hereinabove.

In a further aspect, the present invention provides novel chemical intermediates of the formulae Ar.CH = NR, Ar.CO.$NHR^1$ or $ArCH_2L$ as herein before defined.

Compounds of the formula (I) in which one or more hydroxy groups are protected, for example by benzyl or trityl groups or by an isoproplylidene group are also useful intermediates in the preparation of compounds of the present invention.

The compounds of this invention have biocidal activity, e.g. are toxic to certain living cells which are detrimental to mammals, for example pathogenic organisms and tumours.

This toxicity to pathogenic organisms has been demonstrated, for example, by activity against one or more of the following: viruses (e.g. Herpes simplex 1/vero), fungi (e.g. Candida albicans), protozoa (e.g. Eimeria tenella and Trichomonas vaginalis), bacteria (e.g. Mycoplasma smegmatis and Streptococcus pyogenes), and helminths (e.g. Nippostrongylus brasiliensis and Brugia pahangi). The antitumour activity of compounds of formula I has been demonstrated in a number of recognized screens and primarily by activity against ascitic P388/O leukaemia.

Preferred compounds of the formula (I) are those which have antitumour activity. The activity against ascitic tumours, including P388/O, is evidenced by reduction of tumour cell number in mammals (for example, mice bearing ascitic tumours) and their consequent increase in survival duration as compared to an untreated tumour bearing control group. Antitumour activity is further evidenced by measurable reduction in the size of solid tumours following treatment of mammals with the compounds of this invention compared to the tumours of untreated control tumour bearing animals. Compounds of formula (I) are active against murine tumours such as lymphocytic leukaemia P388/O, lymphocytic leukaemia L1210, melanotic melanoma B16, P815 mastocytoma, MDAY/D2 fibrosarcoma, colon 38 adenocarcinoma, M5076 rhabdomyosarcoma and Lewis lung carcinoma.

Activity in one or more of these tumour tests has been reported to be indicative of antitumour activity in man (A. Goldin et al. in Methods in Cancer Research ed. V.T. DeVita Jr. and H. Busch, 16, 165, Academic Press, N.Y. 1979).

There are sublines of P388/O which have been made resistant to the following clinically useful agents: cytosine arabinoside, doxorubicin, cyclophosphamide, 1-phenylalanine mustard, methotrexate, 5-fluorouracil, actinomycin D, cis-platin and bis-chloroethylnitrosourea. Compounds of this invention show potent activity against these drug-resistant tumours using the procedure for P388/O above.

Compounds of formula (I) have also been found to be active against human tumour cells in primary cultures of lung, ovary, breast, renal, melanoma, unknown primary, gastric, pancreatic, mesothelioma, myeloma, and/or colon cancer. (As used herein "cancer" is to be taken as synonymous with "malignant tumour" or more generally "tumour" unless otherwise noted.) This is a procedure in which the prevention of tumour cell colony formation, i.e. tumour cell replication, by a drug has been shown to correlate with clinical antitumour activity in man (D.D. Von Hoff et al., Cancer Chemotherapy and Pharmacology 6, 265 (1980); S. Salmon and D.D. Von Hoff, Seminars in Oncology, 8, 377 (1981)).

Compounds of formula I which have been found to have antitumour activity intercalate in vitro with DNA (this property is determined by viscometric methods using the procedure of W. D. Wilson et al., Nucleic Acids Research 4, 2697 (1954)) and have a log P as calculated by the method of C. Hansch and A. Leo in Substituent Constants for Correlation Analysis in Chemistry and Biology, John Wiley and Sons, New York, 1979, lying in the range between -2.0 and +2.5.

As has been described above, the compounds of the present invention are useful for the treatment of tumours. The invention thus further provides a method for the treatment of tumours in animals, including mammals, especially humans, which comprises the administration of a clinically useful amount of compound of formula (I) in a pharmaceutically useful form, once or several times a day or other appropriate schedule, orally, rectally, parenterally, or applied topically.

In addition, there is provided as a further, or alternative, aspect of the invention, a compound of formula (I) for use in therapy, for example as an antitumour agent.

The amount of compound of formula (I) required to be effective as a biocidal agent will, of course, vary and is ultimately at the discretion of the medical or veterinary practitioner. The factors to be considered include the condition being treated, the route of administration, and nature of the formulation, the mammal's body weight, surface area, age and general condition, and the particular compound to be administered. A suitable effective antitumour dose is in the range of about 0.1 to about 120 mg/kg body weight, preferably in the range of about 1.5 to 50 mg/kg, for example 10 to 30 mg/kg. The total daily dose may be given as a single dose, multiple doses, e.g., two to six times per day or by intravenous infusion for selected duration. For example, for a 75 kg mammal, the dose range would be about 8 to 9000 mg per day, and a typical dose would be about 2000 mg per day. If discrete multiple doses are indicated, treatment might typically be 500 mg of a compound of formula I given 4 times per day in a pharmaceutically useful formulation.

Whilst it is possible for the active compound (defined herein as compound of formula (I), or ether, ester, or salt thereof) to be administered alone, it is preferable to present the active compound in a pharmaceutical

formulation. Formulations of the present invention, for medical use, comprise an active compound together with one or more pharmaceutically acceptable carriers thereof and optionally other therapeutical ingredients. The carrier(s) must be pharmaceutically acceptable in the sense of being compatible with the other ingredients of the formulation and not deleterious to the recipient thereof.

The present invention, therefore, further provides a pharmaceutical formulation comprising a compound of formula (I) (in the form of the free base, ether, or ester derivative or a pharmaceutically acceptable acid addition salt thereof) together with a pharmaceutically acceptable carrier therefor.

There is also provided a method for the preparation of a pharmaceutical formulation comprising bringing into association a compound of formula (I) an ether, ester, or pharmaceutically acceptable salt thereof, and a pharmaceutically acceptable carrier therefor.

Whilst the antitumour activity of the compounds of formula (I) is believed to reside in the free base, it is often convenient to administer an acid addition salt of a compound of formula (I).

The formulations include those suitable for oral, rectal or parenteral (including subcutaneous, intramuscular and intravenous) administration. Preferred formulations are those suitable for oral or parenteral administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into assocation with a liquid carrier or a finely divided solid carrier or both and then, if necessary, shaping the product into desired formulations.

Formulations of the present invention suitable for oral administration may be presented as discrete units such as capsules, cachets, tablets or lozenges, each containing a predetermined amount of the active compound; as a powder or granules; or a suspension in an aqueous liquid or non-aqueous liquid such as a syrup, an elixir, an emulsion or a draught.

A tablet may be made by compression or moulding, optionally with one or more accessory ingredients. Compressed tablets may be prepared by compressing in a suitable machine the active compound in a free-flowing form such as a powder or granules, optionally mixed with a binder, lubricant, inert diluent, surface active or dispersing agent. Moulded tablets may be made by moulding in a suitable machine, a mixture of the powdered active compound with any suitable carrier.

A syrup may be made by adding the active compound to a concentrated, aqueous solution of a sugar, for example sucrose, to which may also be added any accessory ingredients. Such accessory ingredient(s) may include flavourings, an agent to retard crystallization of the sugar or an agent to increase the solubility of any other ingredient, such as a polyhydric alcohol for example glycerol or sorbitol.

Formulations for rectal administration may be presented as a suppository with a conventional carrier such as cocoa butter.

Formulations suitable for parenteral administration conveniently comprise a sterile aqueous preparation of the active compound which is preferably isotonic with the blood of the recipient. Such formulations suitably comprise a solution of a pharmaceutically and pharmacologically acceptable acid addition salt of a compound of the formula (I) that is isotonic with the blood of the recipient. Thus, such formulations may conveniently contain distilled water, 5% dextrose in distilled water or saline and a pharmaceutically and pharmacologically acceptable acid addition salt of a compound of the formula (I) that has an appropriate solubility in these solvents, for example the hydrochloride, isethionate and methanesulfonate salts, preferably the latter.

Useful formulations also comprise concentrated solutions or solids containing the compound of formula (I) which upon dilution with an appropriate solvent give a solution suitable for parenteral administration as above.

In addition to the aforementioned ingredients, the formulations of this invention may further include one or more accessory ingredient(s) selected from diluents, buffers, flavouring agents, binders, surface active agents, thickeners, lubricants, preservatives (including antioxidants) and the like.

The following examples are provided by the way of illustration of the present invention and should in no way be construed as a limitation thereof.

General Comments

All solvents were reagent grade and used without further purification with the following exceptions. Tetrahydrofuran (THF) was dried by distillation from Na/K alloy under nitrogen ($N_2$) and used immediately. Toluene ($PhCH_3$) was distilled from $CaH_2$ under $N_2$ and stored over 3A molecular sieves. Chemicals used

were reagent grade and used without purification unless noted. The full name and address of the suppliers of the reagents and chemicals is given when first cited. After this, an abbreviated name is used.

Preparative HPLC was carried out on a Waters Prep LC/System 500A machine using two 500 g silica gel ($SiO_2$) cartridges unless otherwise noted. Plugs of $SiO_2$ used for purifications were "flash chromatography" silica gel (Merck Co., Inc., Merck Chemical Division, Rahway, NJ, 07065 silica gel 60, 230-400 mesh). An appropriate volume sintered glass funnel was filled approximately 3/4 full with the $SiO_2$ and packed evenly by tapping the outside of the funnel. A piece of filter paper was then placed on top of the $SiO_2$ and a solution of the material to be purified applied evenly to the top. Gentle suction through a filter flask moved the eluting solvent through the plug rapidly. The appropriate size fractions were combined as needed and further manipulated.

General procedures are described in detail. Analogous procedures show melting point (mp), recrystallization solvents, and elemental analyses (all elements analyzing within a difference of $\pm$ 0.4% of the expected value). Any changes to the procedure such as solvent, reaction temperature, reaction time, or workup are noted.

NMR ($^1H$, $^{13}C$), IR, MS data of all new products were consistent with the expected and proposed structures. The positions assigned to structural isomers were unequivocally determined by a number of NMR techniques. All final products were dried in a vacuum oven at 20 mm Hg pressure at the temperature indicated overnight (12-16 h). All temperatures are in degrees Celsius.

## Example 1

### 2-Methyl-2-(((10-methyl-10H-benzofuro(3,2-b)indol-6-yl)methyl)amino)-1,3-propanediol

#### 1A 2-(2-Nitrophenyl)benzofuran

To a RB flask equipped with a magnetic stirring bar, rubber septum and $N_2$ inlet line was added a solution of benzofuran (Aldrich Chemical Co., P.O. Box 2060, Milwaukee, WI, 53201, 34.26 g, 0.29 mol) in $Et_2O$ (800 ml). A solution of n-BuLi (Aldrich, 20.5 g, 0.32 mol) in hexane (200 ml) was added to the flask via cannula. The mixture was stirred for 30 min. at RT. The mixture was then added via cannula to a RB flask equipped with a magnetic stirring bar, rubber septum and $N_2$ inlet line that contained a solution of 2-fluoronitrobenzene (Aldrich, 37.26 g, 0.264 mol) in $Et_2O$ (800 ml) cooled to -40° ($CH_3CN$/dry ice bath). The resulting mixture was stirred overnight at RT. $H_2O$ (40 ml) was then added to the flask to destroy excess n-BuLi. The mixture was then further diluted with $H_2O$ (1 L), the two layers separated and the $H_2O$ layer extracted with $Et_2O$ (2x500 ml). The organic layers were combined, dried ($Na_2SO_4$) and concentrated to give a dark red oil. This material was passed through a plug of $SiO_2$ using hexane/EtOAc (9:1) as the eluting solvent. The appropriate fractions were combined and the solvent removed to give 34.0 g (49.7% yield) of crude solid that was used in the next step without additional purification. A portion was washed with hexane to give 2-(2-nitrophenyl)benzofuran, mp 79-80.5° (C,H,N).

#### 1B 10H-Benzofuro(3,2-b)indole

To a RB flask equipped with magnetic stirring bar, reflux condenser and $N_2$ inlet line was added 2-(2-nitrophenyl)benzofuran (1A, 70.0 g,0.29 mol) and triethylphosphite (Aldrich, 98.0 g, 0.59 mol). The resulting mixture was heated at 100-110° for 7 h. The mixture was then dissolved in EtOAc (500 ml) and passed through a plug of $SiO_2$ using EtOAc as the eluting solvent. The appropriate fractions were combined and concentrated to give a crude material which was dissolved in a mixture of hexane/EtOAc (1:1) and passed through a plug of $SiO_2$ using hexane/EtOAc (9:1) as the eluting solvent. The appropriate fractions were combined and concentrated to give 53.5 g (89% yield) of crude solid that was used in the next step without additional purification. A portion was recrystallized from EtOAc/hexane (1:6) to give 10H-benzofuro(3,2-b)-indole, mp 197.5-198° (C,H,N).

#### 1C 10-Methyl-10H-benzofuro(3,2-b)indole

To a RB flask equipped with magnetic stirring bar, reflux condenser, rubber septum and $N_2$ inlet line was added a 60% oil dispersion of NaH (Aldrich,5.78 g,0.151 mol). The oil was removed from the dispersion by washing with hexane (3x100 ml). Dry THF (200 ml) was then added to the flask via cannula to cover the NaH. A solution of 10H-benzofuro(3,2-b)indole (1B,28.4 g,0.137 mol) in THF (200 ml) was added to the flask via cannula. The mixture was stirred for 15 min. at RT. Dimethyl sulfate (Aldrich,19.0 g, 0.151 mol) was then

added to the flask by syringe and the resulting mixture was stirred 90 min. at RT. $H_2O$ (10 ml) was added to the flask to destroy excess NaH. The mixture was diluted with 0.1 N HCl (1 L) and extracted with EtOAc (1 L). The organic layer was washed with $H_2O$ (2x1 L), dried ($Na_2SO_4$) and concentrated to give 35.6 g of crude material. This material was passed through a plug of $SiO_2$ using $PhCH_3$ as the eluting solvent. The appropriate fractions were combined and the solvent removed to give after drying 29.0 g (96.0% yield) of 10-methyl-10H-benzofuro-(3,2-b)indole, mp 114-115° (C,H,N).

1D <u>10-Methyl-10H-benzofuro(3,2-b)indole-6-carbaldehyde</u>

To a RB flask equipped with a magnetic stirring bar, rubber septum, and $N_2$ inlet line was added a solution of 10-methyl-10H-benzofuro(3,2-b)indole (1C,10.0 g,44 mmol) in dry THF (1 L). The mixture was cooled to -78°, then a solution of s-BuLi (Aldrich,1.3 M in cyclohexane, 8.8 mmol,6.8 ml) was added by syringe. The resulting mixture was allowed to warm to -30° then recooled to -78° before adding DMF (Aldrich,0.67 g,9.2 mmol) by syringe. The resulting mixture was stirred overnight at RT. $H_2O$ (10 ml) was added to the flask to destroy excess s-BuLi. The mixture was concentrated and the residue was partitioned between EtOAc (1 L) and 0.2 N HCl (1 L). The organic layer was washed with satd. NaCl solution, dried, ($Na_2SO_4$), and concentrated to give a yellow solid which was triturated with $Et_2O$ (300 ml) to yield after drying 9.4 g (86.0%) of 10-methyl-10H-benzofuro(3,2-b)indole-6-carbaldehyde, mp 114.5-115.5° (C,H,N).

1E <u>2-Methyl-2-(((10-methyl-10H-benzofuro(3,2-b)indol-6-yl)methyl) amino)-1,3-propanediol Hydrochloride</u>

To a 3-necked RB flask equipped with a magnetic stirring bar, condenser, thermometer, Dean-Stark trap and $N_2$ inlet line was added 10-methyl-10H-benzofuro(3,2-b)indole-6-carbaldehyde (1D, 9.3 g,37.3 mmol), 2-amino-2-methyl-1,3-propanediol (Aldrich,7.84 g,74.6 mmol), *p*-toluenesulfonic acid monohydrate (Aldrich,0.1 g) and $PhCH_3$ (300 ml). The mixture was stirred at reflux with azeotropic removal of $H_2O$ for 2.5 h. Most of the $PhCH_3$ was then removed by distillation. The mixture was then cooled in an ice bath and diluted with abs. EtOH (300 ml) and further cooled. Solid $NaBH_4$ (Aldrich, 2.82 g,74.6 mmol) was added in one portion to the reaction mixture. The ice bath was then removed, the reaction mixture allowed to warm to RT and stirred overnight. The crude reaction mixture was concentrated to dryness, vigorously stirred with $H_2O$ (500 ml) and filtered. After thorough washing with $H_2O$ and drying, the resulting solid crude free base was converted to its HCl salt by treatment with a 5M solution of g. HCl in absolute EtOH. The solution was filtered through a medium porosity sintered glass funnel and precipitated by addition of $Et_2O$ (final volume 2 L). Recrystallization twice ($CH_3OH/Et_2O$) followed by filtration and drying gave 5.7 g (40.8% yield) of 2-methyl-2-(((10-methyl-10H-benzofuro(3,2-b)indol-6-yl) methyl)amino)-1,3-propanediol hydrochloride, mp 258-258.5°, (C,H,N,Cl).

Additional comments: Azetropic removal of $H_2O$ is continued until complete. This process normally takes 1-4 h. If the crude reaction mixture does not form an easily filterable solid, it can be extracted with EtOAc (2x500 ml). The organic layers are then combined, washed with $H_2O$ (2x500 ml), satd. NaCl solution (2x500 ml), dried ($K_2CO_3$), filtered and the solvent removed to give the solid crude free base which is converted to its HCl salt by the method described above. Normally $CH_3OH$, EtOH and occasionally i-PrOH is used to produce the solution. Alternatively, in cases where the HCl salt is not soluble enough in $H_2O$, the $CH_3SO_3H$ salt is made by treating the free base with 1.1 equivalents of $CH_3SO_3H$(99.5%, Alfa Products, P.O. Box 8247, Ward Hill, MA 01835-0747) dissolved in abs. EtOH (10% v/v) in an appropriate alcohol solvent ($CH_3OH$,EtOH,i-PrOH). After dissolution, the mixture is filtered through a sintered glass frit and diluted with $Et_2O$. The resulting solid is recrystallized once or twice to give the pure final product.

<u>Example 2</u>

<u>2-Methyl-2-(((10-methyl-10H-thieno(3,2-a)carbazol-4-yl)methyl)amino)-1,3-propanediol</u>

2A <u>10-Methyl-10H-thieno(3,2-a)carbazole</u>

Using the procedure outlined in Example 1C, 10H-thieno(3,2-a) carbazole (Cambridge Chemicals, Inc., 202 East Smith Street, Milwaukee, WI 53207) and dimethyl sulfate (Aldrich) gave a 96.0% yield of 10-methyl-10H-thieno(3,2-a)carbazole. This material was used without further purification.

2B <u>10-Methyl-10H-thieno(3,2-a)carbazole-4-carbaldehyde</u>

10-Methyl-10H-thieno(3,2-a)carbazole (2A, 30.0 g,126.mmol) was formylated using the procedure of A. Rieche et al., Chem. Ber. 93, 88 (1960). The crude reaction product was passed through a plug of $SiO_2$ using $CH_2Cl_2$ as the eluting solvent. The appropriate fractions were combined and the solvent removed to give after drying 6.04 g (18% yield) of 10-methyl-10H-thieno (3,2-a)carbazole-4-carbaldehyde.

2C 2-Methyl-2-(((10-methyl-10H-thieno(3,2-a)carbazol-4-yl)methyl) amino)-1,3-propanediol methanesulfonate

Using the procedure outlined in Example 1E, 10-methyl-10H-thieno-(3,2-a)carbazole-4-carbaldehyde (2B) and 2-amino-2-methyl-1,3-propanediol (Aldrich) gave a 44.0% yield of 2-methyl-2-(((10-methyl-10H-thieno(3,2a)carbazol-4-yl)methyl)amino)-1,3-propanediol methanesulfonate, mp 187-188.5°, (EtOH/$Et_2O$), (C,H,N.S).

Example 3

2-Methyl-2-(((10-methyl-10H-thieno(3,2-a)carbazol-2-yl)methyl)amino)-1,3-propanediol

3A 10-Methyl-10H-thieno(3,2-a)carbazole-2-carbaldehyde

Using the procedure outlined in Example 1D, 10-methyl-10H-thieno-(3,2-a)carbazole-2-carbaldehyde (2A,30.75 g,133.8 mmol) was formylated to give 36.92 g of crude solid. Trituration in hot $PhCH_3$ followed by recrystallization from EtOAc/hexane (1:2) gave after drying 10.90 g (30.7% yield) of 10-methyl-10H-thieno-(3,2-a)carbazole-2-carbaldehyde, mp 207-208.5° (C,H,N,S).

3B 2-Methyl-2-(((10-methyl-10H-thieno(3,2-a)carbazol-2-yl)methyl)amino)-1,3-propanediol methanesulfonate

Using the procedure outline in Example 1E, 10-methyl-10H-thieno (3,2-a)carbazole-2-carbaldehyde (3A) and 2-amino-2-methyl-1,3-propanediol (Aldrich) gave a 68.2% yield of 2-methyl-2-(((10-methyl-10H-thieno-(3,2-a)carbazol-2-yl)methyl)amino)-1,3-propanediol methanesulfonate, mp 219-220°, (C,H,N,S).

Example 4

2(((Benzofuro(5,4-b)benzofuran-8-yl)methyl)amino)-2-methyl-1,3-propanediol

4A Ethyl-2-(Dibenzofuranyloxy)acetate

To a 2 L 3-necked RB-flask equipped with mechanical stirrer, reflux condenser, addition funnel and $N_2$ inlet line was added 2-hydroxydibenzofuran (Aldrich,250 g,0.814 mol), $K_2CO_3$ (Mallinckrodt, Inc., 2nd & Mallinckrodt Street, St. Louis, MO 63147,247.61 g,1.79 mol) and dry acetone (500 ml). The mixture was stirred vigorously for 45 min. at RT. $BrCH_2COOEt$ (Aldrich 136 g,0.814 mol) was then added dropwise to the flask over 0.5 h. The mixture was then refluxed for 2.5 h. The mixture was cooled to RT, filtered and the solvent removed from the resulting filtrate to give a dark brown semisolid. This material was stirred vigorously with hot hexane (3 L). The light yellow solution was decanted from a dark brown oil. The solution was decolorized with Norit$^R$, filtered through a Celite$^R$ plug, concentrated to a volume of 2 L and allowed to stand at RT overnight. The off-white crystals that formed were removed by filtration. A second crop of product was obtained from the filtrate after refrigeration overnight. The two crops were combined and dried affording 103 g (46.8% yield) of crude product. This material was used without further purification. Additional crystallation from hexane gave pure ethyl 2-(2-dibenzofuranyloxy)acetate, mp 51.5-53°,(C,H).

4B Ethyl 2-((1-Formyl-2-dibenzofuranyl)oxy)acetate

4C Ethyl 2-((3-Formyl-2-dibenzofuranyl)oxy)acetate

Ethyl 2-(2-dibenzofuranyloxy)acetate (4A,182.0 g, 0.673 mol) was formylated using the procedure of A. Rieche et al., Chem. Ber. 93, 88 (1960). The reaction mixture was passed through a plug of $SiO_2$ using $CH_2Cl_2$ as the eluting solvent affording after combination of the appropriate fractions and removal of solvent 151.3 g of crude material shown to contain two isomeric aldehyde esters by NMR. The mixture was again chromatographed on $SiO_2$ using $CH_2Cl_2$ as the eluting solvent. The fractions containing isomerically pure aldehyde esters were combined. Fractions containing mixtures of the two aldehyde esters were also

combined and rechromatographed until the mixture was completely separated. A total of 71.3 g (35.5% yield) of the faster eluting ($R_f$ = 0.24, $SiO_2/CH_2Cl_2$) aldehyde ester isomer shown by NMR to be the 1-CHO derivative was obtained and used without further purification. Recrystallization (EtOAc) of a portion of this material gave pure ethyl 2-((1-formyl-2-dibenzofuranyl)oxy)acetate, mp 100.5-101.5°, (C,H). A total of 81.2 g (40.4% yield) of the slower eluting aldehyde ester isomer ($R_f$ = 0.14,$SiO_2/CH_2Cl_2$) shown by NMR to be the 3-CHO derivative was obtained and used without further purification. Recrystallization (EtOAc) of a portion of this material gave pure ethyl 2-((3-formyl-2-(dibenzofuranyl)-oxy)-acetate, mp 177-178°, (C,H).

4D Ethyl Benzofuro(5,4-b)benzofuran-2-carboxylate

To a 1 L 3-necked RB flask equipped with mechanical stirrer, reflux condenser, thermometer and $N_2$ inlet line was added ethyl 2-((1-formyl-2-dibenzofuranyl)oxy)acetate (4B,5628 g,0.189 mol), $K_2CO_3$ - (Mallinckrodt, 31.29 g,0.226 mol) and dry DMF (600 ml). The mixture was heated to 150° over 20 min. and then stirred for 1 h. The reaction was cooled to RT and filtered through a fritted glass funnel. The solvent was removed to give 62.1 g of crude brown oil. This material was partitioned between EtOAc (1.5 L) and $H_2O$ (1 L). The EtOAc layers were combined and washed with $H_2O$ (750 ml), satd. NaCl (750 ml) and then dried ($Na_2SO_4$). The mixture was filtered and the solvent removed to give after drying 54.2 g of off-white solid. The solid was dissolved in warm $PhCH_3$ (400 ml) and passed through a plug of $SiO_2$ using $PhCH_3$ as the eluting solvent. The appropriate fractions were combined and the solvent removed to give 35.9 g (61.7%) of ethyl benzofuro(5,4-b)benzofuran-2-carboxylate, mp 176.5-178.5°, (C,H).

4E Ethyl 8-Formyl-benzofuro(5,4-b)benzofuran-2-carboxylate

4F Ethyl 9-Formyl-benzofuro(5,4-b)benzofuran-2-carboxylate 0.1 $CH_2Cl_2$

4G Ethyl 5-Formyl-benzofuro(5,4-b)benzofuran-2-carboxylate

4H Ethyl 4-Formyl-benzofuro(5,4-b)benzofuran-2-carboxylate

Ethyl benzofuro(5,4-b)benzofuran-2-carboxylate (4D,63.2 g, 0.225 mol) was formylated using the procedure of A. Rieche et al., Chem. Ber. 93, 88(1960). The crude reaction product was chromatographed on $SiO_2$ using $CH_2Cl_2$ as the eluting solvent several times until the mixture was resolved into four aldehyde esters:

Ethyl 8-formylbenzofuro(5,4-b)benxofuran-2-carboxylate, 4.64 g (6.7% yield), mp 214-215°, isolated directly from chromatography, (C,H), $R_f$ = 0.62 ($SiO_2/CH_2/Cl_2$).

Ethyl 9-formylbenzofuro(5,4-b)benzofuran-2-carboxylate 0.1 $CH_2$-$Cl_2$,19.8 g (27.7% yield), mp 176-178.5°, isolated directly from chromatography, (C,H), $R_f$ = 0.60 ($SiO_2/CH_2Cl_2$).

Ethyl 5-formylbenzofuro(5,4-b)benzofuran-2-carboxylate, 0.05 g (0.07% yield), mp 201-202.5°, ($PhCH_3$), (C,H) $R_f$ = 0.55 ($SiO_2$/ $CH_2Cl_2$).

Ethyl 4-formylbenzofuro(5,4-b)benzofuran-2-carboxylate, 16.6 g (23.9% yield), mp 225° (dec), ($CHCl_3$), $R_f$ = 0.35 ($SiO_2/CH_2Cl_2$).

4I Ethyl 8-(Hydroxymethyl)benzofuro(5,4-b)benzofuran-2-carboxylate

To 1 L RB flask equipped with magnetic stirring bar and $N_2$ inlet line was added ethyl 8-formylbenzofuro(5,4-b)benzofuran-2-carboxylate (4E,3.70 g,12.0 mmol) and dry THF (600 ml). The mixture was warmed until the starting material was completely dissolved. The solution was cooled to 0° and $NaBH_4$ (Aldrich, 0.45 g,12.0 mmol) was added in one portion. After stirring for 30 mins. at 0°, the reaction mixture was treated with $H_2O$ (300 ml) and acidified to pH - 1 with 1 N HCl. The mixture was extracted with EtOAc (3x500 ml), satd. NaCl solution (3x500 ml) and dried ($NaSO_4$). After filtration the solvent was removed and the resulting white solid dried in a vacuum oven. A total of 3.80 g (~100% yield) of ethyl 8-(hydroxymethyl)-benzofuro(5,4-b)benzofuran-2-carboxylate, mp 172-173°, (C,H) was obtained and used directly in the next step.

4J 8-(Hydroxymethyl)benzofuro(5,4-b)benzofuran-2-carboxylic acid

To a 500 ml RB flask equipped with magnetic stirring bar and $N_2$ inlet line was added ethyl 9-(hydroxymethyl)benzofuro(5,4-b)-benzofuran-2-carboxylate (4I,3.70 g,11.92 mmol), THF (150 ml) and a

solution of 1 N NaOH solution (12 ml,12 mmol) in EtOH (25 ml). The solution turns milky white instantly. After 20 min. $H_2O$ (50 ml) was added to the mixture. The resulting white solid was filtered and washed with $H_2O$ (3x200 ml) and dried overnight in a vacuum oven at 90° after drying 3.17 g (93.2% yield) of 8-(hydroxymethyl)benzofuro(5,4-b)benzofuran-2-carboxylic acid, 0.2 $H_2O$, mp 260-262° (dec), (C,H).

4K Benzofuro(5,4-b)benzofuran-8-methanol

To a 500 ml 3-necked RB flask equipped with magnetic stirring bar, reflux condenser, thermometer and $N_2$ inlet line was added 8-(hydroxymethyl)benzofuran-2-carboxylic acid 0.2 $H_2O$ (4J,3.13 g, 11.1 mmol), $Cu_2O$ (Aldrich, 4.76 g,33.27 mmol) and quinoline (Aldrich, 225 ml). The reaction mixture was warmed to 200-205° and stirred for 30 min. After cooling, the quinoline was removed by distillation leaving a dark green oil. This material was dissolved in $CH_2Cl_2$ (150 ml) and passed through a plug of $SiO_2$ using $CH_2Cl_2$ as the eluting solvent. The appropriate fractions were combined and the solvent removed by rotary evaporation to give after drying, 2.26 g (85.6% yield) of benzofuro(5,4-b)benzofuran-8-methanol, mp 137-138°, (C,H).

4L Benzofuro(5,4-b)benzofuran-8-carbaldehyde

To a 500 ml RB flask equipped with magnetic stirring bar, reflux condenser and $N_2$ inlet line was added benzofuro(5,4-b)benzofuran-8-methanol (4K,2.26 g,0.29 mmol), $BaMnO_4$ (Aldrich, 4.76 g,18.6 mmol) and $CH_2Cl_2$ (300 ml). The mixture was refluxed for 2 h, filtered through a plug of Celite[R] and the solvent removed to give after drying 2.08 g of crude product. This material was dissolved in $CH_2Cl_2$ (50 ml) and passed through a plug of $SiO_2$ using $PhCH_3$ as the eluting solvent. The appropriate fractions were combined and the solvent removed to give 2.06 g of (94.1%) of benzofuro(5,4-b)benzofuran-8-carbaldehyde, mp 156.5-158°, (C,H).

4M 2(((Benzofuro(5,4-b)benzofuran-8-yl)methyl)amino)-2-methyl-1,3-propanediol Hydrochloride

Using the procedure outlined in Example 1E, benzofuro(5,4-b)benzofuran-8-carbaldehyde (4L) and 2-amino-2-methyl-1,3-propanediol (Aldrich) gave a 57.3% yield of 2-(((benzofuro(5,4-b)benzofuran-8-yl)-methyl)amino)-2-methyl-1,3-propanediol hydrochloride, mp 262-263.5°, ($CH_3OH/Et_2O$), (C,H,N,Cl).

Example 5

2-(((Benzofuro(5,4-b)benzofuran-9-yl)methyl)amino)-2-methyl-1,3-propanediol

5A Ethyl 9-(Hydroxymethyl)benzofuro(5,4-b)benzofuran-2-carboxylate

Using the procedure outlined in Example 4I, ethyl 9-formylbenzofuro(5,4-b)benzofuran-2-carboxylate 0.1 $CH_2Cl_2$ gave a 88.9% yield of ethyl 9-(hydroxymethyl)benzofuran(5,4-b)benzofuran-2-carboxylate, mp 138-140°, (C,H).

5B 9-(Hydroxymethyl)benzofuro(5,4-b)benzofuran-2-carboxylic acid

Using the procedure outlined in Example 4J, ethyl 9-(hydroxymethyl)-benzofuro(5,4-b)benzofuran-2-carboxylate (5A) gave a 98.6% yield of 9-(hydroxymethyl)benzofuro(5,4-b)benzofuran-2-carboxylicacid, mp > 295°, (C,H).

5c Benzofuran(5,4-b)benzofuran-9-methanol

Using the procedure outlined in Example 4K, 9-(hydroxymethyl)benzofuro(5,4-b)benzofuran-2-carboxylic acid (5B) gave a 75.9% yield of benzofuro(5,4-b)benzofuran-9-methanol, mp 132-133°, (C,H).

5D Benzofuro(5,4-b)benzofuran-9-carbaldehyde

Using the procedure outlined in Example 4L, benzofuro(5,4-b)benzofuran-9-methanol (5C) gave a 92.3% yield of benzofuro(5,4-b)benzofuran-9-carbaldehyde, mp 183-184°, (C,H).

5E 2-(((Benzofuro(5,4-b)benzofuran-9-yl)methyl)amino)-2-methyl-1,3-propanediol Hydrochloride

Using the procedure outlined in Example 1E, benzofuro(5,4-b)benzofuran-9-carbaldehyde (5D) and 2-amino-2-methyl-1,3-propanediol (Aldrich) gave a 34.2% yield of 2-(((benzofuro(5,4-b)benzofuran-9-yl)-methyl)amino)2-methyl-1,3-propanediol hydrochloride, mp 243-244.5°, (EtOH/$Et_2O$), (C,H,N,Cl).

Example 6

2-(((Benzofuro(5,4-b)benzofuran-4-yl)methyl)amino)-2-methyl-1,3-propanediol

6A 4-Formyl-benzofuro(5,4-b)benzofuran-2-carboxylic acid

Using the procedure outlined in Example 4J, ethyl 4-formylbenzofuro(5,4-b)benzofuran-2-carboxylate (4H) gave a 81.8% yield of 4-formylbenzofuro(5,4-b)benzofuran-2-carboxylic acid which was directly in the next step without further purification.

6B Benzofuro(5,4-b)benzofuran

6C Benzofuro(5,4-b)benzofuran-4-methanol

4-Formyl-benzofuro(5,4-b)benzofuran-2-carboxylic acid was decarboxylated (6A,13.60 g,48.5 mmol) using the procedure outlined for the hydroxymethyl carboxylic acid derivative in Example 4K. Two major products were formed in this reaction and crudely separated by column chromatography ($SiO_2$) using $CH_2Cl_2$ as the eluting solvent. The fractions containing the faster eluting material were collected the solvent removed and the residue chromatographed ($SiO_2$) using $PhCH_3$ as the eluting solvent. The appropriate fractions were combined and the solvent removed to give 2.61 g (25.8% yield) of benzofuro(5,4-b)-benzofuran, mp 66-67°, C,H. The fractions containing the slower eluting material were also combined and the solvent removed to give 3.79 g (32.7% yield) of benzofuro(5,4-b)benzofuran-4-methanol), mp 129-131°, C,H. The aldehyde carboxylic acid starting materials undergoes a Cannizaro type reaction unlike the hydroxymethyl carboxylic acid (4J).

6D Benzofuro(5,4-b)benzofuran-4-carbaldehyde

Using the procedure outlined in Example 4L, benzofuro(5,4-b)benzofuran-4-methanol (6C) gave a 78.6% yield of benzofuro(5,4-b)benzofuran-4-carbaldehyde, mp 170.5-171.5°, (C,H).

6E 2-(((Benzofuro(5,4-b)benzofuran-4-yl)methyl)amino)-2-methyl-1,3-propanediol Hydrochloride

Using the procedure outlined in Example 1E, benzofuro(5,4-b)benzofuran-4-carbaldehyde (6D) and 2-amino-2-methyl-1,3-propanediol (Aldrich) gave a 52.0% yield of 2-(((benzofuro(5,4-b)benzofuran-4-yl)-methyl)amino)-2-methyl-1,3-propanediol hydrochloride, mp 263-265° (dec), ($CH_3OH/Et_2O$), (C,H,N,Cl).

Example 7

2-(((Benzofuro(5,4-b)benzofuran-2-yl)methyl)amino)-2-methyl-1,3-propanediol

7A Benzofuro(5,4-b)benzofuran-2-carbaldehyde

To a 300 ml RB flask equipped with magnetic stirring bar, rubber septum and $N_2$ inlet line was added benzofuro(5,4-b)benzofuran (6B,2.46 g,11.81 mmol), dry THF (125 ml) and N,N,N',N'-tetramethylethylenediamine(Aldrich, 3.16 g,27.17 mmol,4.10 ml) (distilled from $CaH_2$). The mixture was cooled to -78°. s-BuLi (Aldrich, 1.3 M in cyclohexane, 27.17 mmol, 20.9 ml) was added dropwise to the mixture by syringe. The mixture was stirred at -78° for 1 h, warmed to -20° for 45 min. and then recooled to -78°. Dry DMF (Mallinckrodt, 3.20 g,41.3 mmol,3.1 ml) was then added dropwise by syringe to the flask. The mixture was stirred at -78° for 10 min. and then allowed to warm to RT (over 1 h). The reaction was quenched with $H_2O$ (25 ml) and acidified with 1 N HCl. The THF was removed by rotary evaporation and the residue further diluted with $H_2O$ (300 ml). This material was extracted with EtOAc (3x500 ml). The organic layers were combined and washed with $H_2O$ (2x500 ml), satd. NaCl solution (1 L) and dried

($Na_2SO_4$). The solvent was removed to give 2.49 g of yellow solid. This material was chromatographed ($SiO_2$) using $PhCH_3$ as the eluting solvent. The appropriate fractions were combined and the solvent removed to give 1.52 g (54.5% yield) of benzofuro(5,4-b)benzofuran-2-carbaldehyde, mp 193-194.5°, (C,H).

7B 2-(((Benzofuro(5,4-b)benzofuran-2-yl)methyl)amino)-2-methyl-1,3-propanediol hydrochloride

Using the procedure outlined in Example 1E, benzofuro(5,4-b) benzofuran-2-carbaldehyde (7A) and 2-amino-2-methyl-1,3-propanediol (Aldrich) gave a 41.5% yield of 2-(((benzofuro(5,4-b)benzofuran-2-yl)-methyl)amino)-2-methyl-1,3-propanediol hydrochloride, mp 230-230.5° (dec), ($EtOH/Et_2O$), (C,H,N,Cl).

Example 8

2-(((Benzofuro(5,6-b)benzofuran-8-yl)methyl)amino)-2-methyl-1,3-propanediol

8A Ethyl Benzofuro(5,6-b)benzofuran-2-carboxylate

To a 1 L 3-necked RB flask equipped with mechanical stirrer, reflux condenser, thermometer and $N_2$ inlet line was added ethyl 2-((3-formyl-2-dibenzofuranyl)oxy)acetate (4C,67.32 g,0.225 mol), $K_2CO_3$ - (Mallinckrodt, 37.43 g,0.271 mol) and dry DMF (800 ml). The mixture was heated to 155° for 1.5 h. The reaction was cooled to RT and filtered through a fritted glass funnel. The solvent was removed to give 82.1 g of crude brown oil. This material was partitioned between EtOAc (1.5 L) and $H_2O$ (2x750 ml), satd. NaCl (2x1 L) and then dried ($Na_2SO_4$). The mixture was filtered and the solvent removed to give after drying 53.2 g of a yellow solid. The solid was dissolved in warm $PhCH_3$ (550 ml) and passed through a plug of $SiO_2$ using $PhCH_3$ as the eluting solvent. The appropriate fractions were combined and the solvent removed to give 40.4 g (53.2% yield) of material that was used without further purification. Recrystallization of a small amount of material from $PhCH_3$ gave ethyl benzofuro(5,6-b)benzofuran-2-carboxylate, mp 123.5-124.5°, (C,H).

8B Ethyl 8-Formyl-benzofuro(5,6-b)benzofuran-2-carboxylate 0.05 $CH_2Cl_2$

8C Ethyl 4-Formyl-benzofuro(5,6-b)benzofuran-2-carboxylate

Ethyl benzofuro(5,6-b)benzofuran-2-carboxylate (8A, 36.0 g, 0.132 mol) was formylated using the procedure of A. Rieche et al., Chem. Ber. 93, 88(1960). The crude reaction mixture was passed through a plug of $SiO_2$ using $CH_2Cl_2$ as the eluting fraction several times until the mixture was resolved into two aldehyde esters:

Ethyl 8-formylbenzofuro(5,6-b)benzofuran-2-carboxylate 0.05 $CH_2Cl_2$, 6.18 g (15.2% yield), mp 227°, isolated directly from chromatography, (C,H), $R_f$ = 0.23 ($SiO_2/CH_2Cl_2$);

Ethyl 4-formylbenzofuro(5,6-b)benzofuran-2-carboxylate, 26.2 g (64.4% yield), ($PhCH_3$), (C,H) $R_f$ = 0.63 ($SiO_2/CH_2Cl_2$).

8D Ethyl 8-(Hydroxymethyl)benzofuro(5,6-b)benzofuran-2-carboxylate

Using the procedure outlined in Example 4I, ethyl 8-formyl-benzofuro(5,6-b)benzofuran (8C) gave a 93.3% yield of ethyl 8-(hydroxymethyl)benzofuro(5,6-b)benzofuran-2-carboxylate, mp 186-186.5%, (C,H).

8E 8-(Hydroxymethyl)benzofuro(5,6-b)benzofuran-2-carboxylic acid

Using the procedure outlined in Example 4J, ethyl 8-(hydromethyl)benzofuro(5,6-b)benzofuran-2-carboxylate (8D) gave a 92.7% yield of 8-(hydroxymethyl)benzofuro(5,6-b)benzofuran-2-carboxylic acid, mp >270°, (C,H).

8F Benzofuro(5,6-b)benzofuran-8-methanol

Using the procedure outlined in Example 4K, 8-(hydroxymethyl)benzofuro(5,6-b)benzofuran-2-carboxylic acid (8E) gave a 74.8% yield of benzofuro(5,6-b)benzofuran-8-methanol, mp = 170.172°, (C,H).

8G Benzofuro(5,6-b)benxofuran-8-carbaldehyde

Using the procedure outlined in Example 4L, benzofuro(5,6-b)benzofuran-8-methanol gave a 89.7% yield of benzofuro(5,6-b)benzofuran-8-carbaldehyde, mp 188-189°, (C,H).

8H 2(((Benzofuro(5,6-b)benzofuran-8-yl)methyl)amino)-2-methyl-1,3-propanediol Hydrochloride

Using the procedure outlined in Example 1E, benzofuro(5,6-b)benzofuran-8-carbaldehyde (8G) and 2-amino-2-methyl-1,3-propanediol (Aldrich) gave a 66.3% yield of 2-(((benzofuro(5,6-b)benzofuran-8-yl)-methyl)amino)-2-methyl-1,3-propanediol hydrochloride, mp 235-236.5° (dec), ($CH_3OH/Et_2O$), (C,H,N,Cl).

Example 9

2-(((Benzofuro(5,6-b)benzofuran-4-yl)methyl)amino)-2-methyl-1,3-propanediol

9A 4-Formyl-benzofuro(5,6-b)benzofuran-2-carboxylic acid

Using the procedure outlined in Example 4J, ethyl 4-formyl-benzofuran-2-carboxylic (8C) gave a 98.9% yield of 4-formyl-benzofuro(5,6-b)benzofuran-2-carboxylic acid, that was used directly in the next step without further purification.

9B Benzofuro(5,6-b)benzofuran

9C Benzofuro(5,6-b)benzofuran-4-methanol

4-Formyl-benzofuro(5,6-b)benzofuran-2-carboxylic acid (9A,25.2 g, 81.7 mmol) using the procedure outlined for the hydroxymethyl carboxylic acid derivative in Example 4K. The two major products formed in this reaction were crudely separated by column chromatography ($SiO_2$) using $CH_2Cl_2$ as the eluting solvent. The fractions containing the faster eluting material were collected, the solvent removed and the residue chromatographed ($SiO_2$) using $PhCH_3$ as the eluting solvent. The appropriate fractions were combined and the solvent removed to give 6.20 g (33.4% yield) of benzofuro(5,6-b)furan, mp 90-100°, (C,H). The fractions containing the slower eluting material were also combined and the solvent removed to give 3.91 g (18.4% yield) of benzofuro(5,6-b)benzofuran-4-methanol, mp 152-153°, (C,H). As mentioned for Examples 6B and 6C, the aldehyde carboxylic acid starting material undergoes a Cannizaro type reaction unlike the hydroxymethyl carboxylic acid 8E.

9D Benzofuro(5,6-b)benzofuran-4-carbaldehyde

Using the procedure outlined in Example 4K, benzofuro(5,6-b)benzofuran-4-methanol (9C) gave a 96.5% yield of benzofuro(5,6-b)benzofuran-4-carbaldehyde, mp 167.5-169°, (C,H).

9E 2-(((Benzofuro(5,6-b)benzofuran-4-yl)methyl)amino)-2-methyl 1,3-propanediol Hydrochloride

Using the procedure outlined in Example 1E, benzofuro(5,6-b)benzofuran-4-carbaldehyde (9D) and 2-amino-2-methyl-1,3-propanediol (Aldrich) gave a 46.9% yield of 2-(((benzofuro(5,6-b)benzofuran-4-yl)-methyl)amino)-2-methyl-1,3-propanediol hydrochloride, mp 224-225° (dec), ($EtOH/Et_2O$), (C,H,N,Cl).

Example 10

2-(((Benzofuro(5,6-b)benzofuran-2-yl)methyl)amino)-2-methyl-1,3-propanediol

10A Benzofuro(5,6-b)benzofuran-2-carbaldehyde

Using the procedure outlined in Example 7A, benzofuro(5,6-b)benzofuran (9B) gave a 82.8% yield of benzofuro(5,6-b)benzofuran-2-carbaldehyde, mp 194-195.5°, (C,H).

10B 2-(((Benzofuro(5,6-b)benzofuran-2-yl)methyl)amino)-2-methyl-1,3-propanediol Hydrochloride

Using the procedure outlined in Example 1E, benzofuro(5,6-b-) benzofuran-2-carbaldehyde (10A) and 2-amino-2-amino-2-methyl-1,3-propanediol (Aldrich) gave a 50.3% yield of 2-(((benzofuro(5,6-b)benzofuran-2-yl)methyl)amino)-2-methyl-1,30-propanediol hydrochloride, mp 231-232.5° (dec), ($CH_3OH/Et_2O$), (C,H,N,Cl).

Example 11

2-Methyl-2-(((1-methyl-1H-[1]benzothieno[2,3-g]indol-3-yl)methyl)amino)-1,3-propanediol

11A Ethyl 1-Methyl-1H[1]benzothieno[2,3-g]indole-2-carboxylate

Using the procedure outlined in Example 1C, ethyl 1H[1]benzotheino-[2,3-g]indole-2-carboxylate (H.G. Pars Pharmaceutical Laboratories, Inc., 763 Concord Ave., Cambridge, MA 02138) and dimethyl sulfate (Aldrich) gave a quantitative yield of ethyl 1-methyl-1H-[1]benzothieno[2,3-g]-indole-2-carboxylate, mp 85-86°, ($PhCH_3$), (C,H,N).

11B Ethyl 3-Formyl-2-methyl-1H[1]benzothieno[2,3-g]indole-2-carboxylate

Ethyl 1-methyl-1H-[1]benzotheino[2,3-g]indole-2-carboxylate (11A) was formylated by the procedure of A. Rieche et al., Chem. Ber. 93, 88(1960) to give a 58.7% yield of ethyl 3-formyl-1-methyl-1H-[1]-benzothieno[2,3-g]indole-2-carboxylate, mp 145-147°, ($CH_2Cl_2$/ hexane), (C,H,N).

11C 3-Formyl-1-methyl-1H-[1]benzothieno[2,3-g]indole-2-carboxylic acid

Using the procedure outlined in Example 4J, ethyl 3-formyl-1-methyl-1H-[1]benzothieno[2,3-g]indole-2-carboxylate (11B) gave a 91.0% yield of 3-formyl-1-methyl-1H-[1]benzothieno[2,3-g]indole-2-carboxylic acid, which was used directly without further purification.

11D 1-Methyl-1H-[1]-benzothieno[2,3-g]indole-3-carbaldehyde

Using the procedure outlined in Example 4K, 3-formyl-1-methyl-1H-[1]-benzothieno[2,3-g]indole-2-carboxylic acid (11C) gave a 35.3% yield of 1-methyl-1H-benzothieno[2,3-g]indole-3-carbaldehyde, mp 192-194°, ($CH_2Cl_2$/petroleum ether), (C,H,N).

11E 2-Methyl-2-(((1-methyl-1H-[1]benzothieno[2,3]indol-3-yl) methyl)amino)-1,3-propanediol Hydrochloride

Using the procedure outlined in Example 1E, 1-methyl-1H-[1]benzothieno-[2,3-g]-indole-3-carbaldehyde (11D) and 2-amino-2-methyl-1,3-propanediol (Aldrich) gave a 56.7% yield of 2-methyl-2-(((1-methyl-1H-[1]-benzothieno[2,3-g]indol-3-yl)methyl)amino)-1,3-propanediol hydrochloride, mp 228-230° (dec), ($CH_3OH/Et_2O$), (C,H,N,Cl).

Example 12

2-(((6-Ethyl-1,6-dihydro-1-methylpyrrolo[3,2-c]carbazol-3-yl)methyl)amino)-2-methyl-1,3-propanediol

12A Ethyl 6-Ethyl-1,6-dihydro-1-methylpyrrolo[3,2-c]carbazole-2-carboxylate

Using the procedure outlined in Example 1C, ethyl 6-ethyl-1,6-dihydro-pyrrolo[3,2-c]carbazole-2-carboxylate (H.G. Pars Pharmaceutical Laboratories, Inc.) gave a 90.5% yield of ethyl 6-ethyl-1,6-dihydro-1-methylpyrrolo[3,2-c]carbazole-2-carboxylate, mp 115.5-116°, ($CH_2Cl_2$/hexane), (C,H,N).

12B 6-Ethyl-1,6-dihydro-1-methylpyrrolo[3,2-c]carbazole-3-carbaldehyde

Using the procedures outlined in Examples 4H, 4J and 4K, ethyl 6-ethyl-1,6-dihydro-2-methylpyrrolo-[3,2-c]carbazole-2-carboxylate (12A) gave a 9.3% yield of 6-ethyl-1,6-dihydro-1-methylpyrrolo[3,2-c]-carbazole-3-carbaldehyde, mp 144-146°, ($CH_2Cl_2$), (C,H,N).

12C 2-(((6-Ethyl-1,6-dihydro-1-methylpyrrolo[3,2-c]carbazol-3-yl) methyl)-amino)-2-methyl-1,3-propanediol Hydrochloride 0.6 $H_2O$

Using the procedure in Example 1E, 6-ethyl-1,6-dihydro-1-methyl pyrrolo[3,2-c]carbazole-3-carbaldhyde

(12B) and 2-amino-2-methyl-1,3-propanediol (Aldrich) gave a 48.3% yield of 2-[[6-ethyl-1,6-dihydro-1-methylpyrrolo[3,2-c]carbazol-3-yl)methyl]amino]-2-methyl-1,3-propanediol hydrochloride 0.6 $H_2O$, mp 265-267°, (EtOH/$Et_2O$), (C,H,N,Cl).

Example 13

2-Methyl-2-(((1-methyl-1H-benzofuro[2,3-g]indol-3-yl)methyl)-amino)-1,3-propanediol

13A Ethyl 1-Methyl-1H-benzofuro[2,3-g]indole-2-carboxylate

Using the procedure outlined in Example 1C, ethyl 1H-benzofuro[2,3-g]indole-2-carboxylate (H.G. Pars Pharmaceutical Laboratories, Inc.) gave a 86.3% yield of ethyl 1-methyl-1H-benzofuro[2,3-g]indole-2-carboxylate, mp 114-116°, (EtOAc), (C,H,N).

13B Ethyl 3-Formyl-1-methyl-1H-benzofuro[2,3-g]indole-2-carboxylate

Ethyl 1-methyl-1H-benzofuro[2,3-g]indole-2-carboxylate (13A) was formylated according to the procedure of A. Rieche et al., Chem. Ber. 93, 88(1960) to give and 82.5% yield of ethyl 3-formyl-1-methyl-1H-benzofuro[2,3-c]-indole-2-carboxylate, mp 190-192°, ($CH_2Cl_2$/hexane), (C,H,N).

13C 3-Formyl-1-methyl-1H-benzofuro[2.3-g]indole-2-carboxylic acid

Using the procedure outlined in Example 4J, ethyl 3-formyl-1-methyl-1H-benzofuro[2,3-g]indole carboxylate (13B) gave a 90.3% yield of 3-formyl-1-methyl-1H-benzofuro[2,3-g]indole-2-carboxylic acid, which was used directly without further purification.

13D 1-Methyl-1H-benzofuro[2.3-g]indole-3-carbaldehyde

Using the procedure outlined in Example 4K, 3-formyl-1-methyl-1H-benzofuro[2,3-g]indole-2-carboxylic acid (13C) gave a 40.1% yield of 1-methyl-1H-benzofuro[2,3-g]indole-3-carbaldehyde, mp 163-165°, ($CH_2$,$Cl_2$/petroleum ether), (C,H,N).

13E 2-Methyl-2-(((1-methyl-1H-benzofuro[2,3-g]indole-3-yl)methyl) amino)-1,3-propanediol Hydrochloride

Using the procedure outlined in Example 1E, 1-methyl-1H-benzofuro[2,3-g]-indole-3-carbaldehyde (13D) and 2-amino-2-methyl-1,3-propanediol (Aldrich) gave a 63.9% yield of 2-methyl-2-(((1-methyl-1H-benzofuro-[2,3-g]indol-3-yl)methyl)amino)-1,3-propanediol hydrochloride, mp 234-236°, (EtOH/$Et_2O$), (C,H,N,Cl).

Example 14

2-Methyl-2-(((3-methyl-3H-[1]benzothieno[2,3-e]indol-1-yl)-methyl) amino)-1,3-propanediol)

14A 3-Methyl-3H-[1]benzothieno[2,3-e]indole-1-carbaldehyde 0.25 $H_2O$

Using the procedure outlined in Example 1C, ethyl 3H-[1]benzothieno[2,3-e]indole-2-carboxylate (H.G. Pars Pharmaceutical Laboratories, Inc.) and dimethyl sulfate (Aldrich) gave a nearly quantitative yield of crude ethyl 3-methyl-3H-[1]benzothieno[2,3-e]indole-2-carboxylate. This material was formylated using the procedure of A. Rieche et al., Chem. Ber. 93, 88(1960) to give crude mixture of aldehydes which was hydrolyzed and decarboxylated without purification using the procedure outlined in Example 15B to give a 19.0% yield of 3-methyl-3H-[1]benzothieno[2,3-e]indole-1-carbaldehyde 0.25 $H_2O$, mp 223.5-224°, ($CH_2Cl_2$/hexane),(C,H,N).

14B 2-Methyl-2-(((3-methyl-3H-[1]benzothieno[2,3-e]indol-1-yl)methyl) amino)-1,3-propanediol Hydrochloride

Using the procedure described in Example 1E, 3-methyl-3H-[1]-benzothieno[2,3-e]indole-1-carbaldehyde (14A) and 2-amino-2-methyl-1,3-propanediol (Aldrich) gave a 41.4% yield of 2-methyl-2-(((3-methyl-3H-[1]benzothieno[2,3-e]indol-1-yl)methyl)amino)-1,3-propanediol hydrochloride, mp 228-230°, ($CH_3OH$/$Et_2O$), (C,H,N,Cl).

Example 15

2-(((10-Methyl-10H-[1]benzothieno[3,2-b]indol-6-yl)methyl)amino)-2-methyl-1,3-propanediol

15A 10-Methyl-10H-[1]benzothieno[3,2-b]indole

Using the procedure outlined in Example 1C, 10H-[1]benzothieno[3,2-b]indole (prepared by the method of K.E. Chippendale and B. Iddon, JCS Perkin Trans. I 2023 (1972) gave and dimethyl sulfate (Aldrich) a 91.9% yield of 10-methyl-10H[1]benzothieno[3,2-b] indole, mp 175-176°, benzofuro(5,6-b)benzofuran-2-carbaldehyde.

15B 10-Methyl-10H-[1]benzothieno[3,2-b]indole-6-carbaldehyde 0.2 $H_2O$

15C 10-Methyl-10H-[1]benzothieno[3,2-b]indole-3-carbaldehyde

10-Methyl-10H-[1]benzothieno[3,2-b]indole (15A) was formylated according to the procedure of A. Rieche et al., Chem. Ber. 93, 88 (1960) to give a low yield of a mixture of two aldehydes. Chromatography ($SiO_2$/$PhCH_3$) gave a 6.7% yield of 10-methyl-10H-[1]benzothieno-[3,2-b]indole-6-carbaldehyde 0.1 $H_2O$ (15B), mp 128-130°, ($CH_2Cl_2$/hexane), (C,H,N,S), $R_f$ = 0.39 ($SiO_2$/$PhCH_3$) and a 22.3% yield of 10-methyl-10H-[1]benzothieno[3,2-b]indole-3-carbaldehyde (15C), mp 188-189.5°, ($PhCH_3$/hexane), (C,H,N,S), $R_f$ = 0.11 ($SiO_2$/$PhCH_3$).

15D 2-(((10-Methyl-10H-[1]benzothieno[3,2-b]indol-6-yl)methyl)amino)-2-methyl-1,3-propanediol Hydrochloride

Using the procedure outlined in Example 1E, 10-methyl-10H-[1]benzothieno(3,2-b]indole-6-carbaldehyde (15B) and 2-amino-2-methyl-1,3-propanediol (Aldrich) gave a 26.9% yield of 2-(((10-methyl-10H-[1]-benzothieno-[3,2-b]indol-6-yl)methyl)amino)-2-methyl-1,3-propanediol hydrochloride, mp 249.5-250°, ($CH_3OH$/ $Et_2O$), (C,H,N,S,Cl).

Example 16

2-(((10-Methyl-10H-[1]benzothieno[3,2-b]indol-3-yl)methyl)amino)-2-methyl-1,3-propanediol hydrochloride

Using the procedure outlined in Example 1E, 10-methyl-10H-[1]benzothieno[3,2-b]indole-3-carbaldehyde (15C) and 2-amino-2-methyl-1,3-propanediol (Aldrich) gave a 39.4% yield of 2-(((10-methyl-10H-[1]-benzothieno[3,2-b]indol-3-yl)methyl)amino)-2-methyl-1,3-propanediol hydrochloride, mp 285.5-259.5°, ($CH_3OH$/$ET_2O$), (C,H,N,S,Cl).

Antitumor Screening Results

Methods for evaluating the antitumor activity of these compounds are essentially those used in the Tumour Panel by the Development Therapeutics Program, Division of Cancer Treatment, National Cancer Institute, A. Goldin et al., Methods in Cancer Research, Vol. XVI, p.165, Academic Press (1979). Some modifications, in dose level and schedule have been made to increase the testing efficiency.

Lymphocytic Leukemia P388/0 Test

$CD2-F_1$ mice, of the same sex weighing within 20+3g, are used for this test. Control and test animals are injected intraperitoneally with a suspension of $10^6$ viable P388/O tumour cells on day 0. In each test several dose levels which bracket the $LD_{20}$ for the compound are evaluated; each dose level group contains 6 animals. The test compounds are prepared either in physiologic saline containing 0.05% Tween 80 or distilled water containing 5% dextrose and are administered intraperitoneally on days, 1, 5, and 9 relative to tumor implant. Doses are on a mg/kg basis according to individual animals' body weights. the day of death for each animal is recorded, the median identified for each group and the ratios of median survival time for treated (T)/control (C) groups are calculated. The criterion for activity is T/C x 100 ≧120%. Results of P388/O testing are summarized in Table I below.

TABLE I

Activity of Compounds Against P388 Lymphocytic Leukemia

| Compounds of Formula | $LD_{20}$ (mg/kg) | Optimal Dose (mg/kg) | % T/C* | 30-Day Survivors |
|---|---|---|---|---|
| 1E | 150 | 250 | 210 | 0/6 |
| 2C | 375 | 350 | 195 | 0/6 |
| 3B | 375 | 275 | 195 | 0/6 |
| 4M | >450 | 550 | 117 | 0/6 |
| 5E | 175 | 150 | 117 | 0/6 |
| 6E | 60 | 45 | 213 | 1/6 |
| 7B | 175 | 175 | 138 | 0/6 |
| 8H | 300 | 300 | 200 | 0/6 |
| 9E | 75 | 75 | 192 | 0/6 |
| 10B | >675 | 600 | 100 | 0/6 |
| 11E | 250 | 300 | 135 | 0/6 |
| 12C | 100 | 100 | 113 | 0/6 |
| 13E | 115 | 100 | 120 | 0/6 |
| 14B | 150 | 125 | 215 | 0/6 |
| 15D | 225 | 300 | 140 | 5/6 |
| 16 | 250 | 250 | 170 | 0/6 |

*Excluding 30 day survivors

Formulation Examples

A. TABLET

| | |
|---|---|
| Compound of Formula I (as hydrochloride) | 500.0 mg |
| Pregelatinised Corn Starch | 60.0 mg |
| Sodium Starch Glycolate | 36.0 mg |
| Magnesium Stearate | 4.0 mg |

The Compound of formula (I) is finely ground and intimately mixed with the powdered excipients, pregelatinised corn starch and sodium starch glycolate. The powders are wetted with purified water to form granules. The granules are dried and mixed with the magnesium stearate. The formulation is then compressed into tablets weighing approximately 600 mg each.

B. TABLET

| | |
|---|---|
| Compound of formula (I) | 500.0 mg |
| Corn Starch | 70.0 mg |
| Lactose | 83.8 mg |
| Magnesium Stearate | 4.2 mg |
| Polyvinylpyrrolidone | 14.0 mg |
| Stearic Acid | 28.0 mg |

The Compound of formula (I) is finely ground and intimately mixed with the powdered excipients, corn starch and lactose. The powders are wetted with a solution of polyvinylpyrrolidone dissolved in purified water and denatured alcohol to form granules. The granules are dried and mixed with the powdered stearic acid and magnesium stearate. The formulation is then compressed into tablets weighing approximately 700 mg each.

C. CAPSULES

| | |
|---|---|
| Compound of formula (I) | 500.0 mg |
| Corn Starch | 50.0 mg |
| Magnesium Stearate | 3.0 mg |

The finely divided compound of formula (I) is mixed with powdered corn starch and wetted with denatured alcohol to densify the powder. The dried powder is mixed with stearic acid and filled into hard-shell gelatin capsules.

D. SYRUP

| | | |
|---|---|---|
| Compound of formula (I) | | 250.0 mg |
| Ethanol | | 250.0 mg |
| Glycerin | | 500.0 mg |
| Sucrose | | 3,500.0 mg |
| Flavouring Agent | | q.s. |
| Colouring Agent | | q.s. |
| Preserving Agent 0.1% | | |
| Purified Water | q.s. to | 5.0 ml |

The Compound of formula (I) is dissolved in the ethanol, glycerin, and a portion of the purified water. The sucrose and preserving agent are dissolved in another portion of hot purified water, and then the colouring agent is added and dissolved. The two solution are mixed and cooled before the flavouring agent is added. Purified water is added to final volume. The resulting syrup is throughly mixed.

## E. IV INJECTION

| | |
|---|---|
| Compound of formula (I) | 5.0 mg |
| Glycerin | q.s. for isotonicity |
| Preservative | 0.1% |
| Hydrochloric Acid or Sodium Hydroxide | as needed for pH adjustment |
| Water for Injection | q.s. to 1 ml |

The compound of formula (I) and preservative is added to the glycerin and a portion of the water for injection. The pH is adjusted with hydrochloric acid or sodium hydroxide. Water for injection is added to final volume and solution is complete after thorough mixing. The solution is sterilised by filtration through a 0.22 micrometer membrane filter and aseptically filled into sterile 10 ml ampoules or vials.

**Claims**

1. A compound of the formula (I)

$ArCH_2NHR$ (I)

or a monomethyl or monoethyl ether thereof, the compound of formula (I) including these ethers containing no more than 29 carbon atoms in total, or an ester or a salt thereof; wherein Ar is a fused tetracyclic hetero aromatic ring system of the formula:

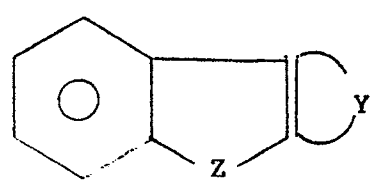

in which Z is oxygen, sulphur or a group $NR^1$ wherein $R^1$ is hydrogen, methyl or ethyl and

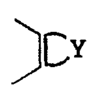

is a bicyclic aromatic ring system comprising a phenyl ring and a 5-membered ring system which contains one heteroatom $Z^1$ selected from oxygen, sulphur or a group $NR^2$ wherein $R^2$ is hydrogen, methyl or ethyl; the tetracyclic ring system being optionally substituted by one or two substituents; said substituents containing not more than four carbon atoms in total when taken together and are the same or different each being selected from halogen; cyano; $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $S(O)_nR^3$ wherein n is an integer 0,1 or 2 and $R^3$ is $C_{1-2}$ alkyl optionally substituted by hydroxy or $C_{1-2}$ alkoxy; or Ar is optionally substituted by a group $NR^4R^5$ containing not more than 5 carbon atoms wherein $R^4$

and $R^5$ are the same or different and each is a $C_{1-3}$ alkyl group or $NR^4R^5$ forms a five-or six-membered heterocyclic ring optionally containing one or two additional heteroatoms;

R contains not more than eight carbon atoms and is a group

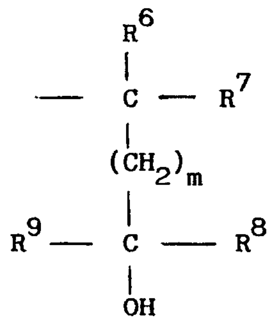

or

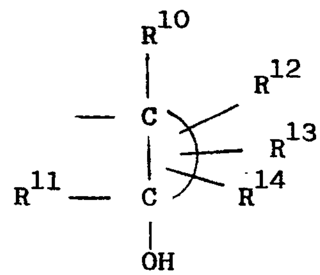

wherein m is 0 or 1;
$R^6$ is hydrogen or $C_{1-3}$ alkyl optionally substituted by hydroxy;
$R^7$ and $R^8$ are the same or different and each is hydrogen or $C_{1-3}$ alkyl;
$R^9$ is hydrogen, methyl or hydroxymethyl;

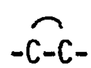

is a five-or six-membered saturated carbocyclic ring;
$R^{10}$, $R^{11}$ and $R^{12}$ are the same or different and each is hydrogen or methyl;

$R^{13}$ is hydrogen, methyl, hydroxy, or hydroxymethyl; $R^{14}$ is hydrogen, methyl, hydroxy or hydroxymethyl.

2. A compound according to claim 1 in which the tetracyclic aromatic ring system Ar is unsubstituted.

3. A compound of the formula (I) according to either claim 1 or 2 in which R is a group:

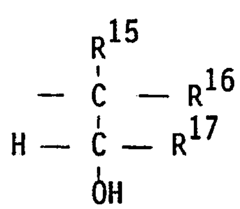

or

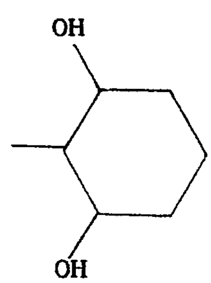

wherein $R^{15}$ is $CH_2OH$, $CH(CH_3)OH$ or $CH_2CH_2OH$,
$R^{16}$ is hydrogen, $C_{1-3}$ alkyl or $CH_2OH$, and
$R^{17}$ is hydrogen or methyl.

4. A compound of the formula (I) according to any one of claims 1 to 3 in which R is a group:

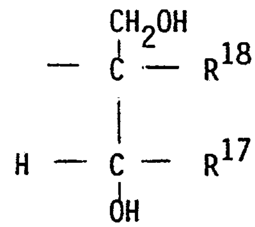

wherein $R^{17}$ is hydrogen or methyl and $R^{18}$ is hydrogen, methyl or ethyl.

5. A compound of the formula (I) according to any one of claims 1 to 4 in which Ar is a group:

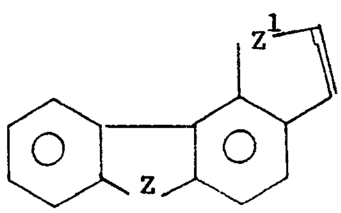

,

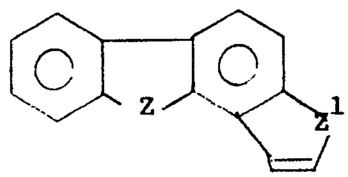

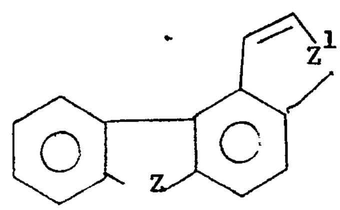

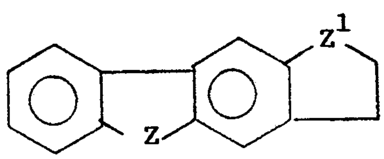

or

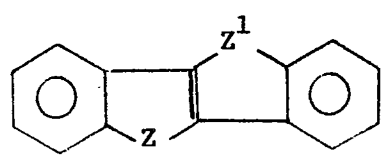

6. A compound of the formula (I) according to any one of claims 1 to 5 selected from

2-(((10-Methyl-10H-[1]benzothieno[3,2-b]indol-3-yl)methyl)amino)-2-methyl-1,3-propanediol,
2-(((10-Methyl-10H-[1]benzothieno[3,2-b]indol-6-yl)methyl)amino)2- methyl-1,3-propanediol,
2-Methyl-2-(((10-methyl-10H-benzofuro(3,2-b)indol-6-yl)methyl) amino)1,3-propanediol,
2-(((Benzofuro(5,6-b)benzofuran-4-yl)methylamino)-2-methyl-1,3-propanediol,

2-(((Benzofuro(5,6-b)benzofuran-8-yl)methyl)amino)-2-methyl-1,3-propanediol,
2-(((Benzofuro(5,6-b)benzofuran-2-yl)methyl)amino)-2-methyl-1,3-propanediol,
2-(((Benzofuro(5,4-b)benzofuran-4-yl)methyl)amino)-2-methyl-1,3-propanediol,
2-(((Benzofuro(5,4-b)benzofuran-9-yl)methyl)amino)-2-methyl-1,3-propanediol,
2-(((Benzofuro(5,4-b)benzofuran-8-yl)methyl)amino)-2-methyl-1,3-propanediol,
2-(((Benzofuro(5,4-b)benzofuran-2-yl)methyl)amino-2-methyl-1,3-propanediol, and monomethyl or monethyl ethers, esters, and addition salts thereof.

7. A process for the preparation of a compound of the formula (I) according to any one of claims 1 to 6 which comprises:
   (i) the reduction of a compound Ar-CH=NR (II) or an appropriately protected derivative thereof followed by deprotection where appropriate.
   (ii) the reduction of a compound ArCO.NHR or a derivative thereof in which the hydroxy groups are protected, followed by deprotection where appropriate, or
   (iii)the reaction of a compound $ArCH_2L$ with a compound $NH_2R$ wherein L is a leaving group; wherein Ar and R are as hereinbefore defined.

8. A novel chemical intermediate involved in the preparation of a compound of the formula (I).

9. A compound of the formula (I) according to any one of claims 1 to 6 for use in medicine.

10. A pharmaceutical formulation which comprises a compound of the formula (I) according to claim 1 together with a pharmaceutically acceptable carrier thereof.

**Claims for the following Contracting States; GR,ES**

1. A process for the preparation of a compound of the formula (I)

$ArCH_2NHR$ (I)

or a monomethyl or monoethyl ether thereof, the compound of formula (I) including these ethers containing no more than 29 carbon atoms in total, or an ester or a salt thereof;
wherein Ar is a fused tetracyclic hetero aromatic ring system of the formula:

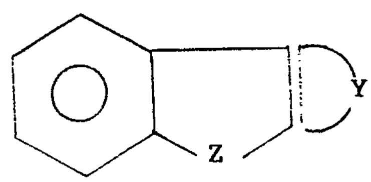

in which Z is oxygen, sulphur or a group $NR^1$ wherein $R^1$ is hydrogen, methyl or ethyl and

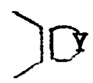

is a bicyclic aromatic ring system comprising a phenyl ring and a 5-membered ring system which contains one heteroatom $Z^1$ selected from oxygen, sulphur or a group $NR^2$ wherein $R^2$ is hydrogen, methyl or ethyl; the tetracyclic ring system being optionally substituted by one or two substituents; said substituents containing not more than four carbon atoms in total when taken together and are the same or different each being selected from halogen; cyano; $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $S(O)_nR^3$ wherein n is an integer 0,1 or 2 and R is $C_{1-2}$ alkyl optionally substituted by hydroxy or $C_{1-2}$ alkoxy; or Ar is optionally substituted by a group $NR^4R^5$ containing not more than 5 carbon atoms wherein $R^4$ and $R^5$ are the same or different and each is a $C_{1-3}$ alkyl group or $NR^4R^5$ forms a five-or six-membered heterocyclic ring optionally containing one or two additional heteroatoms;

R contains not more than eight carbon atoms and is a group

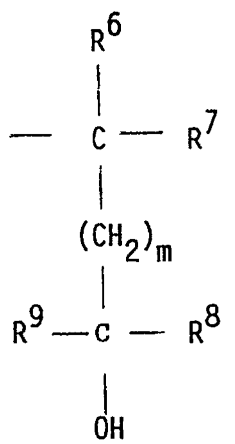

or

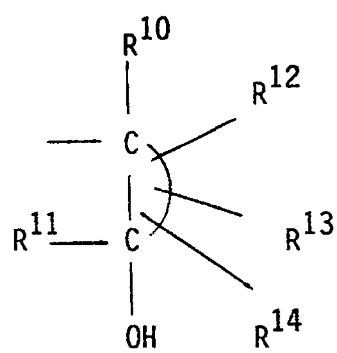

wherein m is 0 or 1;
$R^6$ is hydrogen or $C_{1-3}$ alkyl optionally substituted by hydroxy;

$R^7$ and $R^8$ are the same or different and each is hydrogen or $C_{1-3}$ alkyl;
$R^9$ is hydrogen, methyl or hydroxymethyl;

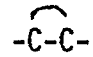

is a five-or six-membered saturated carbocyclic ring;
$R^{10}$, $R^{11}$ and $R^{12}$ are the same or different and each is hydrogen or methyl;

$R^{13}$ is hydrogen, methyl, hydroxy, or hydroxymethyl; $R^{14}$ is hydrogen, methyl, hydroxy or hydroxymethyl,

which comprises:

(i) the reduction of a compound Ar-CH=NR (II) or an appropriately protected derivative thereof followed by deprotection where appropriate.
(ii) the reduction of a compound ArCO.NHR or a derivative thereof in which the hydroxy groups are protected, followed by deprotection where appropriate, or
(iii)the reaction of a compound $ArCH_2L$ with a compound $NH_2R$ wherein L is a leaving group; wherein Ar and R are as hereinbefore defined.

2. A process according to claim 1 for the preparation of a compound in which the tetracyclic aromatic ring system Ar is unsubstituted.

3. A process according to either claim 1 or 2 for the preparation of a compound of the formula (I) according to either claim 1 or 2 in which R is a group:

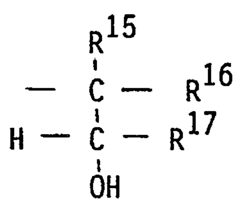

or

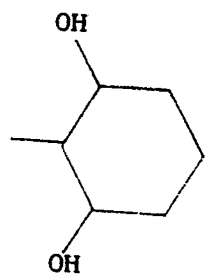

wherein $R^{15}$ is $CH_2OH$, $CH(CH_3)OH$ or $CH_2CH_2OH$,
$R^{16}$ is hydrogen, $C_{1-3}$ alkyl or $CH_2OH$, and
$R^{17}$ is hydrogen or methyl.

4. A process according to any one of claims 1 to 3 for the preparation of a compound of the formula (I) in which R is a group:

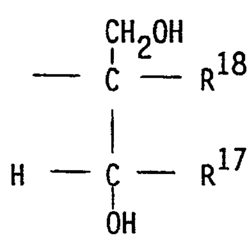

wherein $R^{17}$ is hydrogen or methyl and $R^{18}$ is hydrogen, methyl or ethyl.

5. A process according to any one of claims 1 to 4 for the preparation of a compound of the formula (I) according to any one of claims 1 to 4 in which Ar is a group:

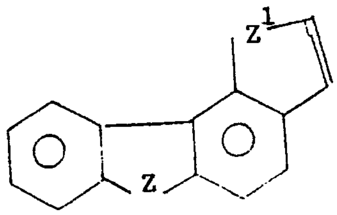

,

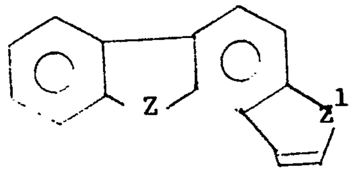

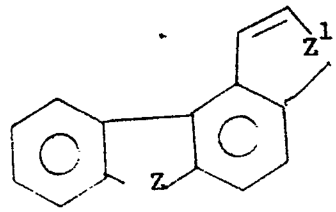

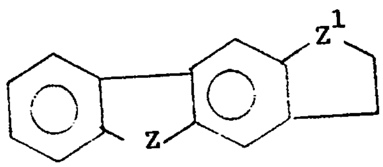

or

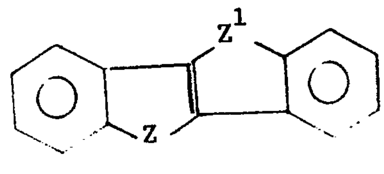

6. A process according to any one of claims 1 to 5 for the preparation of a compound of the formula (I) selected from

2-(((10-Methyl-10H-[1]benzothieno[3,2-b]indol-3-yl)methyl)amino)-2-methyl-1,3-propanediol,
2-(((10-Methyl-10H[1]benzothieno[3,2-b]indol-6-yl)methyl)amino)-2- methyl-1,3-propanediol,
2-Methyl-2-(((10-methyl-10H-benzofuro(3,2-b)indol-6-yl)methyl) amino)1,3-propanediol,
2-(((Benzofuro(5,6-b)benzofuran-4-yl)methylamino)-2-methyl-1,3-propanediol,

2-(((Benzofuro(5,6-b)benzofuran-8-yl)methyl)amino)-2-methyl-1,3-propanediol,
2-(((Benzofuro(5,6-b)benzofuran-2-yl)methyl)amino)-2-methyl-1,3-propanediol,
2-(((Benzofuro(5,4-b)benzofuran-4-yl)methyl)amino)-2-methyl-1,3-propanediol,
2-(((Benzofuro(5,4-b)benzofuran-9-yl)methyl)amino)-2-methyl-1,3-propanediol,
2-(((Benzofuro(5,4-b)benzofuran-8-yl)methyl)amino)-2-methyl-1,3-propanediol,
2-(((Benzofuro(5,4-b)benzofuran-2-yl)methyl)amino-2-methyl-1,3-propanediol, and monomethyl or monethyl ethers, esters, and addition salts thereof.

7. A novel chemical intermediate involved in the preparation of a compound of the formula (I).

8. A compound of the formula (I) according to any one of claims 1 to 6 for use in medicine.

9. A pharmaceutical formulation which comprises a compound of the formula (I) according to any one of claims 1 to 6 together with a pharmaceutically acceptable carrier thereof.

10. The use of a compound of the formula (I) according to any one of claims 1 to 6 in the manufacture of a medicament for use in medicine.

European Patent Office

# PARTIAL EUROPEAN SEARCH REPORT

which under Rule 45 of the European Patent Convention shall be considered, for the purposes of subsequent proceedings, as the European search report

Application number: EP 90 30 3008

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 182 608 (WELLCOME)<br>* Claims 1,2; pages 17-20 *<br>---- | 1,10 | C 07 D 491/044<br>C 07 D 495/04<br>C 07 D 493/04<br>C 07 D 487/04<br>A 61 K 31/40<br>A 61 K 31/38<br>A 61 J 31/34//<br>(C 07 D 491/044<br>C 07 D 307:00<br>C 07 D 209:00)<br>(C 07 D 495/04<br>C 07 D 333:00<br>C 07 D 209:00)<br>(C 07 D 493/04<br>C 07 D 307:00<br>C 07 D 307:00)<br>(C 07 D 487/04<br>C 07 D 209:00<br>C 07 D 209:00)<br>./. |

TECHNICAL FIELDS SEARCHED (Int. Cl.4)

C 07 D 491/00
C 07 D 495/00
C 07 D 493/00
C 07 D 487/00
A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7,9,10
Claims searched incompletely:
Claims not searched: 8
Reason for the limitation of the search:

The intermediate to which claim 8 refers are not defined.

| Place of search | Date of completion of the search | Examiner |
|---|---|---|
| THE HAGUE | 21-11-1990 | ALFARO |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# PARTIAL EUROPEAN SEARCH REPORT

Application number

EP 90 30 3008

-2-

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| | | | (C 07 D 495/04<br>C 07 D 333:00<br>C 07 D 209:00) |
| | | | TECHNICAL FIELDS SEARCHED (Int Cl 4) |
| | | | |

## ANNEX TO THE EUROPEAN SEARCH REPORT ON EUROPEAN PATENT APPLICATION NO.

EP 90 30 3008

This annex lists the patent family members relating to the patent documents cited in the above-mentioned European search report. The members are as contained in the European Patent Office EDP file on 06/12/90
The European Patent Office is in no way liable for these particulars which are merely given for the purpose of information.

| Patent document cited in search report | Publication date | Patent family member(s) | Publication date |
|---|---|---|---|
| EP-A- 0182608 | 28-05-86 | AU-B- 590527 | 09-11-89 |
| | | AU-A- 4993085 | 22-05-86 |
| | | CA-A- 1256114 | 20-06-89 |
| | | JP-A- 2085252 | 26-03-90 |
| | | JP-A- 61158961 | 18-07-86 |

EPO FORM P0459

For more details about this annex : see Official Journal of the European Patent Office, No. 12/82